# EUROPEAN PATENT APPLICATION

(11) **EP 4 123 015 A1**
(43) Date of publication of application: **25.01.2023**
(21) Application number: 21772329.5
(22) Date of filing: 18.03.2021
(51) Int. Cl.: C12N 5/077, A61K 35/34, A61L 27/38, A61P 9/10

(54) **CARDIOMYOCYTE PURIFICATION METHOD**

(30) Priority: 19.03.2020 JP 2020050268
(71) Applicant: Orizuru Therapeutics, Inc., Fujisawa-shi Kanagawa 251-8555 (JP)
(72) Inventor: YOSHIDA, Yoshinori, Kyoto-shi, Kyoto 606-8501 (JP); WATANABE, Kozo, Fujisawa-shi, Kanagawa 251-0012 (JP); TANAKA, Aya, Fujisawa-shi, Kanagawa 251-0012 (JP)
(74) Representative: J A Kemp LLP
(86) International application number: PCT/JP2021/011231
(87) International publication number: WO 2021/187601

(57) **Abstract**

A method for producing a cell population containing cardiomyocytes, including (1) a step of bringing a receptor-type tyrosine kinase inhibitor (excluding EGF receptor inhibitors) into contact with a cell population containing cardiomyocytes or cardiac progenitor cells, and other cells, the cell population being obtained by culturing pluripotent stem cells in a medium for cardiomyocyte differentiation, and (2) a step of culturing the cell population is provided by the present invention.

## Description

### [Technical Field]

The present invention relates to a production method and a purification method of cardiomyocytes. More particularly, the present invention relates to a production method and a purification method of cardiomyocytes by using a receptor-type tyrosine kinase inhibitor.

### (Background of the Invention)

Although the incidence of myocardial infarction has been decreasing in recent years, cardiac diseases including myocardial infarction still remain the leading cause of death worldwide. Heart transplantation is currently the only treatment for patients with severe heart failure, but the shortage of donors poses a problem for heart transplantation. Therefore, cell therapy using cardiomyocytes is attracting attention as a therapeutic method that improves heart disease. In addition, establishment of an in vitro drug efficacy evaluation test or drug safety test using cardiomyocytes is also attracting attention. Therefore, a stable supply of uniform cardiomyocytes that can be used for cell therapy and in vitro tests is required.

One of the methods for stably providing homogeneous cardiomyocytes is to induce the differentiation of cardiomyocytes from stem cells or cardiomyocyte progenitor cells, and various efforts have been made to establish an efficient method for inducing differentiation into cardiomyocytes. As such differentiation induction method, a method for promoting differentiation induction from pluripotent stem cells into cardiomyocytes by culturing pluripotent stem cells in a medium containing an EGFR inhibitor (Patent Literature 1), a method for inducing differentiation of induced pluripotent stem cells into cardiomyocytes and then maturing the cardiomyocytes by contacting the cardiomyocytes with a Neuregulin 1 antagonist or ErbB antagonist (Patent Literature 2), a method for promoting differentiation induction of undifferentiated progenitor cells such as myoblasts by contacting the undifferentiated progenitor cells with a deacetylase inhibitor (Patent Literature 3), a method for promoting differentiation induction of adult progenitor cells such as myocardial progenitor cells and the like by contacting the progenitor cells with a histone deacetylase (HDAC) inhibitor (Patent Literature 4), and the like have been reported. In addition, a method that does not undergo a process of differentiation induction from stem cells has also been reported. For example, Patent Literature 5 discloses a method for producing myocardial progenitor cells or cardiomyocytes from somatic cells such as fibroblasts and the like by direct reprogramming.

### [Citation List]

### [Patent Literature]

[PTL 1]
   WO 2014/136519
[PTL 2]
   US-A-2010/0183565
[PTL 3]
   WO 2003/033678
[PTL 4]
   WO 2009/073618
[PTL 5]
   WO 2015/038704

### [Summary of Invention]

### [Technical Problem]

An object of the present invention is to provide a method for producing a cell population containing high-purity cardiomyocytes by means different from the above-mentioned conventional methods. Another object of the present invention is to provide a method for purifying cardiomyocytes at high purity from a cell population containing cardiomyocytes.

### [Solution to Problem]

The present inventors have conducted intensive studies in an attempt to solve the above-mentioned problems and acquired an idea that, in a cell population already containing cardiomyocytes, the ratio of cardiomyocytes in the cell population may be increased, in other words, cardiomyocytes may be purified, by suppressing the proliferation of cells other than cardiomyocytes or reducing the number of cells other than cardiomyocytes, by inhibitors targeting proteins highly expressed in cells other than cardiomyocytes, rather than promoting differentiation induction from undifferentiated cells into cardiomyocytes. Therefore, first using a cell population containing cardiomyocytes induced to differentiate from iPS cells, single cell RNA sequence analysis was performed to cluster the cells in the cell population. As a result of clustering, it was found that the expression of receptor-type tyrosine kinases is different between cardiomyocytes and other cells. Thus, changes in the ratio of cardiomyocytes in the cell population was examined using inhibitors against receptor-type tyrosine kinase, and it was found that cardiomyocytes can be successfully purified by using a receptor-type tyrosine kinase inhibitor. The present inventors have conducted further studies based on these findings and completed the present invention.

Accordingly, the present invention provides the following.
[1] A method for producing a cell population comprising cardiomyocytes, comprising
   (1) a step of bringing a receptor-type tyrosine kinase inhibitor (excluding EGF receptor inhibitors) into contact with a cell population containing cardiomyocytes or cardiac progenitor cells, and other cells, the cell population being obtained by culturing pluripotent stem cells in a medium for cardiomyocyte differentiation, and
   (2) a step of culturing the cell population.
[2] The method of [1], wherein the cell population is contacted with the receptor-type tyrosine kinase inhibitor in the aforementioned step (1) on or after 4 days from the start of differentiation induction of pluripotent stem cells.
[3] The method of [1] or [2], wherein the cell population is contacted with the receptor-type tyrosine kinase inhibitor for not less than one day in the aforementioned step (1).
[4] The method of any of [1] to [3], wherein the aforementioned inhibitor is at least one inhibitor against a receptor-type tyrosine kinase selected from the group consisting of a VEGF receptor, a PDGF receptor, an HGF receptor, and an FGF receptor.
[5] The method of any of [1] to [4], wherein the aforementioned inhibitor is at least one member selected from the group consisting of N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, N-{4-[(6,7-dimethoxyquinolin-4-yl)oxy]-3-fluorophenyl}-N'-(4-fluorophenyl)cyclopropane-1,1-dicarboxamide, AMG337, ASP5878, BGJ398, Foretinib, ZM323881, CP-673451, Crenolanib, and Crizotinib.
[6] The method of any of [1] to [5], wherein the aforementioned pluripotent stem cell is an induced pluripotent stem cell.
[7] A cell population containing cardiomyocytes, obtained by the method of any of [1] to [6].
[8] An agent for cell transplantation therapy, comprising the cell population of [7].
[9] A method for purifying cardiomyocytes, comprising
   (1) a step of bringing a receptor-type tyrosine kinase inhibitor into contact with a cell population containing cardiomyocytes or cardiac progenitor cells, and other cells, the cell population being obtained by culturing pluripotent stem cells in a medium for cardiomyocyte differentiation, and
   (2) a step of culturing the cell population.

### [Advantageous Effects of Invention]

According to the present invention, a cell population containing cardiomyocytes at a high purity is provided. Such cell population can be preferably used for cell transplantation therapy for cardiac diseases. In addition, a method for purifying cardiomyocytes at a high purity from a cell population containing cardiomyocytes and cardiac progenitor cells is also provided.

### [Brief Description of Drawings]

[Fig. 1]

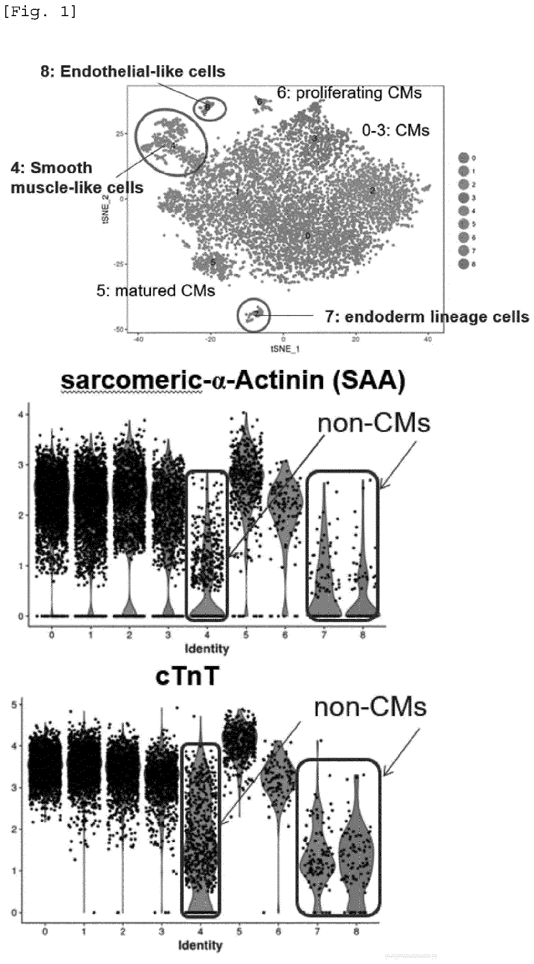

   Fig. 1 shows a t-SNE plot of clustering and the expression levels of sarcomeric-α-actinin and cTnT (Cardiac Troponin T) of each cluster, based on single RNA sequencing data of cardiomyocytes induced to differentiate from iPS cells. The circled portions in the upper panel show non-cardiomyocyte populations. The vertical axis of the center and bottom panels shows the gene expression level and the Identity label of the horizontal axis shows the number of the cluster of Fig. 1. Each dot shows an individual cell, and the framed part shows the cluster of non-cardiomyocytes.
[Fig. 2-1]

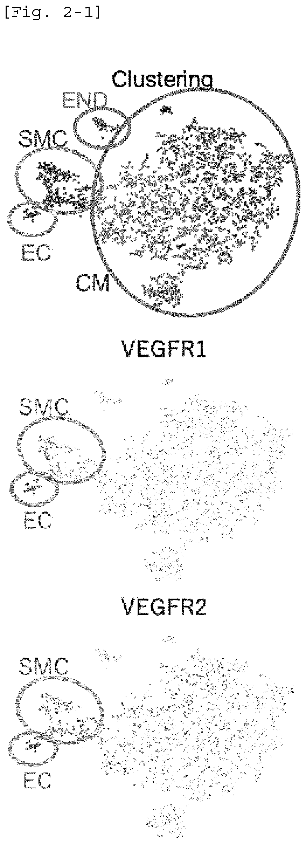

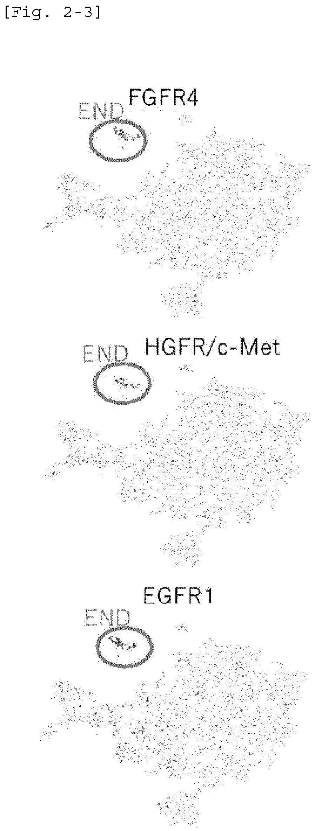

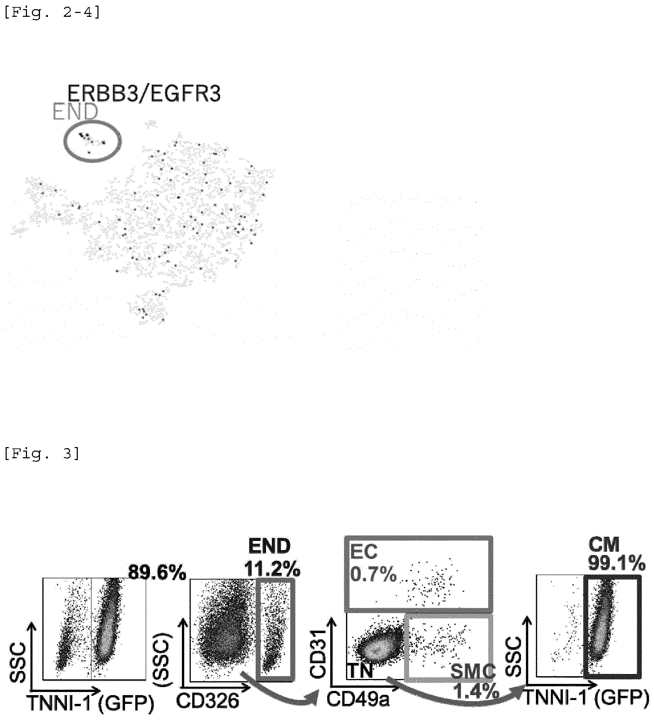

   Fig. 2-1 shows a t-SNE plot of the clustering results of iPS cell-derived cardiomyocytes and the expression of VEGFR1 and VEGFR2 genes in each cell, based on single RNA sequencing data. In the panels showing the gene expression, dark gray dots show cells with high expression level and light gray dots show cells with low expression level. Cell populations with high expression are circled and the population names are indicated in the Figure (CM: cardiomyocytes (hereinafter CM), SMC: smooth muscle-like cells (hereinafter SMC), END: endoderm lineage cells (hereinafter END), EC: endothelium-like cells).
[Fig. 2-2]

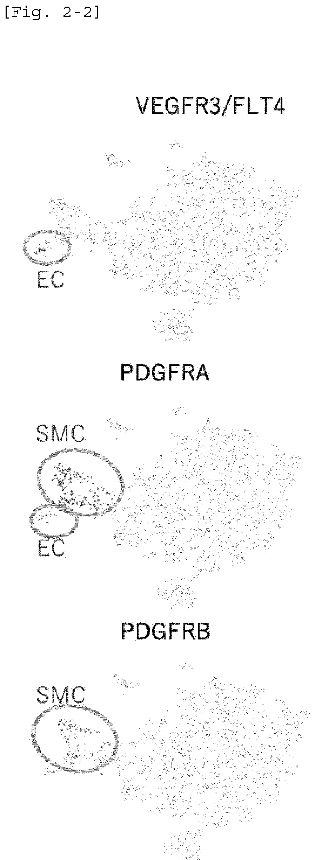

   Fig. 2-2 shows a t-SNE plot of the clustering results of iPS cell-derived cardiomyocytes and the expression of VEGFR3, PDGFRA, and PDGFRB genes in each cell, based on single RNA sequencing data. In the panel showing the gene expression, dark gray dots show cells with high expression level and light gray dots show cells with low expression level. Cell populations with high expression are circled and population names are indicated in the Figure (SMC: smooth muscle-like cells (hereinafter SMC), EC: endothelial-like cells).
[Fig. 2-3]
   Fig. 2-3 shows a t-SNE plot of the clustering results of iPS cell-derived cardiomyocytes and the expression of FGFR4, HGFR (c-Met), and EGFR1 genes in each cell, based on single RNA sequencing data. In the panel showing the gene expression, dark gray dots show cells with high expression level and light gray dots show cells with low expression level. Cell populations with high expression are circled and population names are indicated in the Figure (END: endoderm lineage cells (hereinafter END)).
[Fig. 2-4]
   Fig. 2-4 shows a t-SNE plot of the clustering results of iPS cell-derived cardiomyocytes and the expression of an EGFR3 gene in each cell, based on single RNA sequencing data. In the panel showing the gene expression, dark gray dots show cells with high expression level and light gray dots show cells with low expression level. Cell population with high expression is circled and population name is indicated in the Figure (END: endoderm lineage cells (hereinafter END)).
[Fig. 3]
   Fig. 3 shows the separation results of the cell population of iPS cell-derived cardiomyocytes based on CD326, CD31, and CD49a expressions. By flow cytometry, TNNI1 reporter iPS cell-derived cardiomyocytes having a cardiomyocyte rate (TNNI1-positive rate) of 89.6% (left end panel) were separated into CD326-positive cells (END, cells framed in the center left panel), CD326-negative CD31-positive cells (EC, cells framed in the center right panel), CD326-negative CD31-negative CD49a-positive cells (SMC, cells framed in the center right panel), and CD326-negative CD31-negative CD49a-negative cells (Triple Negative: TN, cells located in the lower left of the center right panel). 99% or more of TN were cardiac muscle marker TNNI1-positive cells (cardiomyocytes) (right end panel). The percentage indications of EC and SMC show not the cells in the panel but the numerical values also including CD326-negative cells.
[Fig. 4]

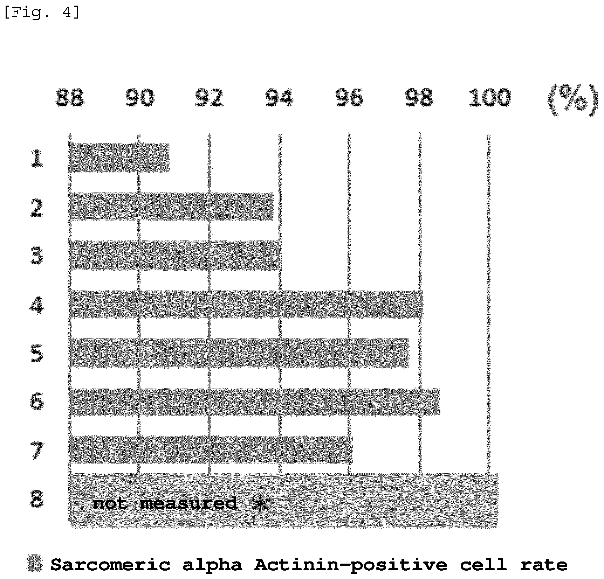

   Fig. 4 shows cardiomyocyte rate after treatment with compound. *: not measured due to low cell count. The vertical axis shows experiment number, and the horizontal axis shows Actinin-positive cell rate (cardiomyocyte rate).
[Fig. 5]

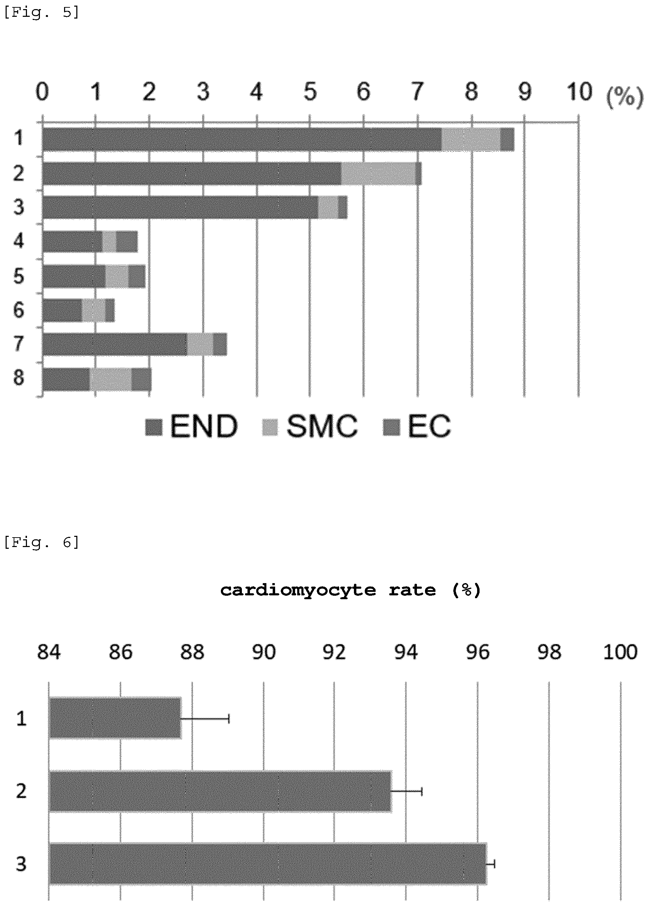

   Fig. 5 shows non-cardiomyocyte rate after treatment with compound (END: endoderm lineage cell, SMC: smooth muscle-like cell, EC: endothelial-like cell). The vertical axis shows experiment number, and the horizontal axis shows non-cardiomyocyte rate.
[Fig. 6]
   Fig. 6 shows mean cardiomyocyte rate after treatment with compound (standard deviation) (n=4). The vertical axis shows experiment number, and the horizontal axis shows cardiomyocyte rate.
[Fig. 7]

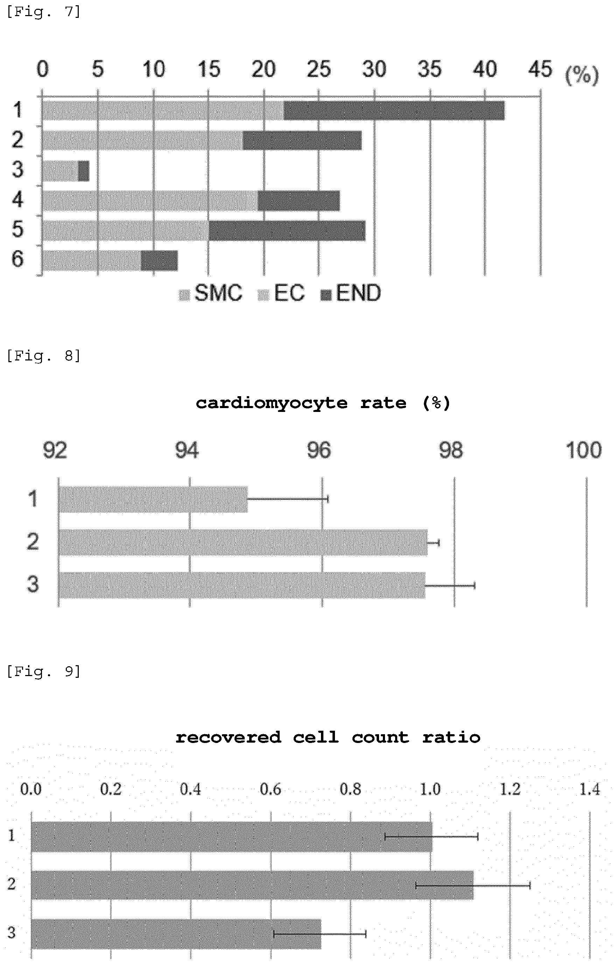

   Fig. 7 shows non-cardiomyocyte rate after treatment with compound (END: endoderm lineage cell, SMC: smooth muscle-like cell, EC: endothelial-like cell). The vertical axis shows experiment number, and the horizontal axis shows non-cardiomyocyte rate.
[Fig. 8]
   Fig. 8 shows mean cardiomyocyte rate after treatment with compound (standard deviation) (n=3). The vertical axis shows experiment number, and the horizontal axis shows cardiomyocyte rate.
[Fig. 9]
   Fig. 9 shows mean relative ratio of recovered cell count after treatment with compound wherein the recovered cell count without treatment with compound is 1 (standard deviation) (n=3). The vertical axis shows experiment number, and the horizontal axis shows relative ratio of recovered cell count.
[Fig. 10]

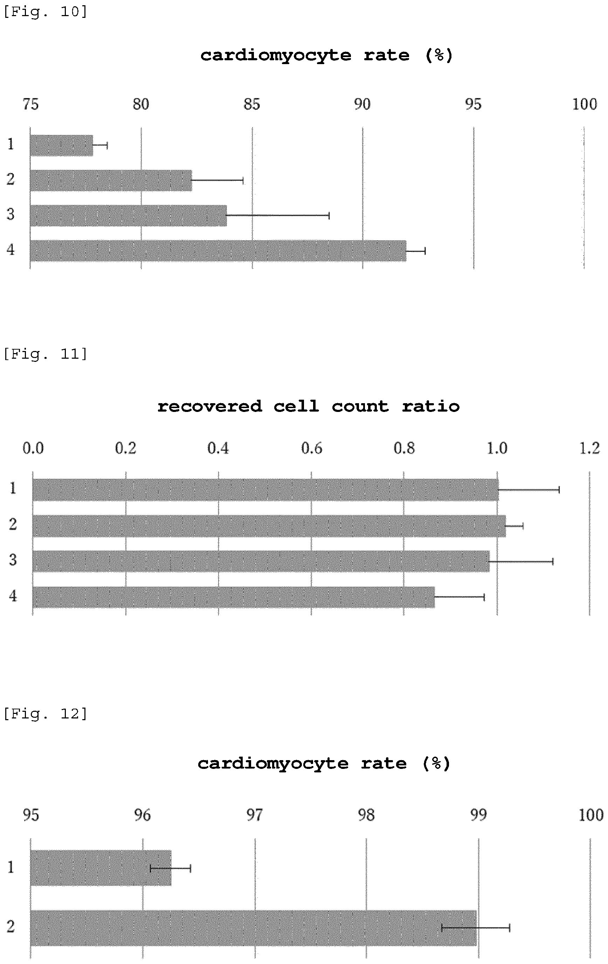

   Fig. 10 shows mean cardiomyocyte rate after treatment with compound (standard deviation) (n=4). The vertical axis shows experiment number, and the horizontal axis shows cardiomyocyte rate.
[Fig. 11]
   Fig. 11 shows mean relative ratio of recovered cell count after treatment with compound wherein the recovered cell count without treatment with compound is 1 (standard deviation) (n=4). The vertical axis shows experiment number, and the horizontal axis shows relative ratio of recovered cell count.
[Fig. 12]
   Fig. 12 shows mean cardiomyocyte rate after treatment with compound (standard deviation) (n=4). The vertical axis shows experiment number, and the horizontal axis shows cardiomyocyte rate.
[Fig. 13]

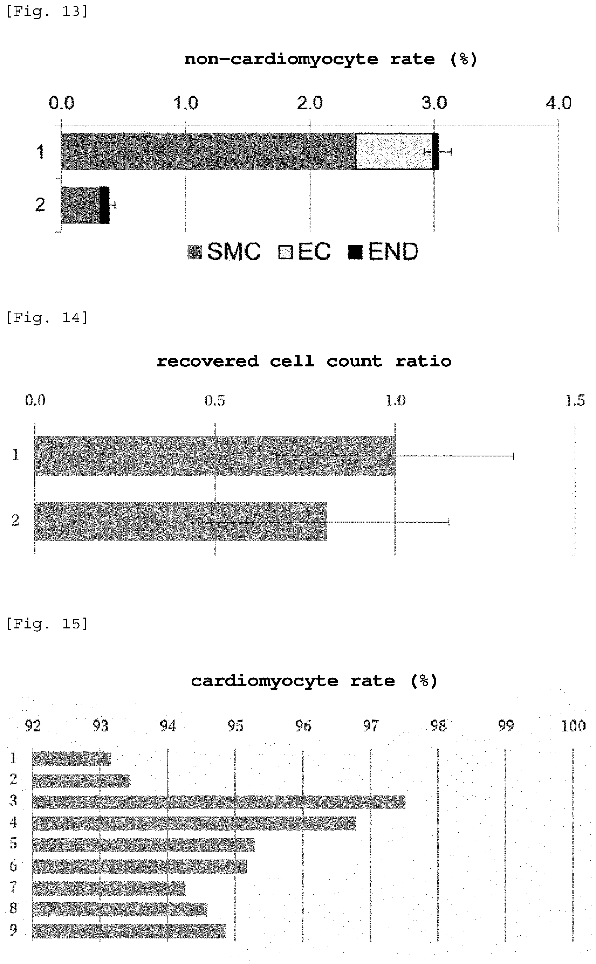

   Fig. 13 shows mean non-cardiomyocyte rate after treatment with compound (standard deviation) (n=4) (END: endoderm lineage cell, SMC: smooth muscle-like cell, EC: endothelial-like cell). The vertical axis shows experiment number, and the horizontal axis shows non-cardiomyocyte rate.
[Fig. 14]
   Fig. 14 shows mean relative ratio of recovered cell count after treatment with compound wherein the recovered cell count without treatment with compound is 1 (standard deviation) (n=4). The vertical axis shows experiment number, and the horizontal axis shows relative ratio of recovered cell count.
[Fig. 15]
   Fig. 15 shows cardiomyocyte rate after treatment with compound (experiment number 1 is mean of n=2, n=1 in others). The vertical axis shows experiment number, and the horizontal axis shows cardiomyocyte rate.
[Fig. 16]

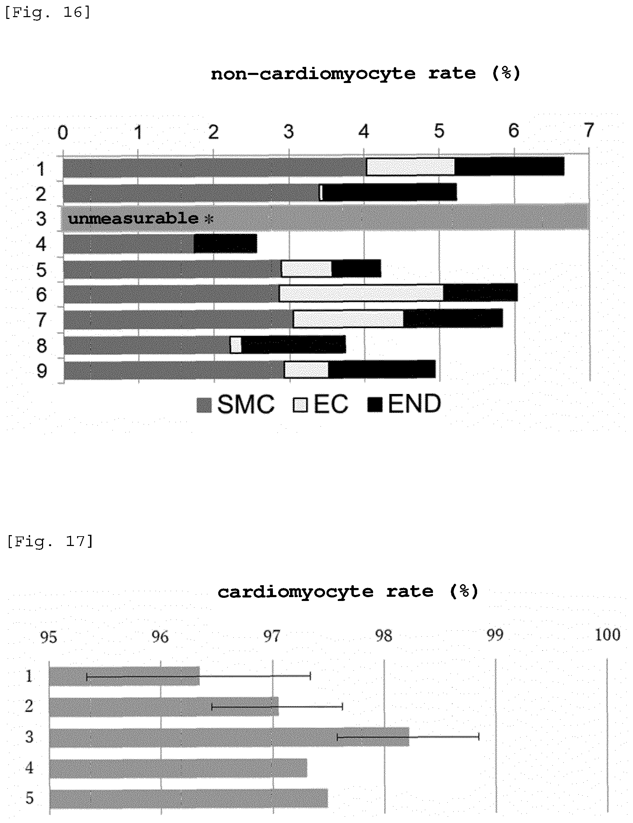

   Fig. 16 shows non-cardiomyocyte rate after treatment with compound (END: endoderm lineage cell, SMC: smooth muscle-like cell, EC: endothelial-like cell). The vertical axis shows experiment number, and the horizontal axis shows non-cardiomyocyte rate. *: not measured due to low cell count.
[Fig. 17]
   Fig. 17 shows cardiomyocyte rate after treatment with compound (experiment number 1 is mean of n=4, experiment numbers 2, 3 are mean of n=3 (standard deviation), n=1 in experiment numbers 4, 5). The vertical axis shows experiment number, and the horizontal axis shows cardiomyocyte rate.
[Fig. 18]

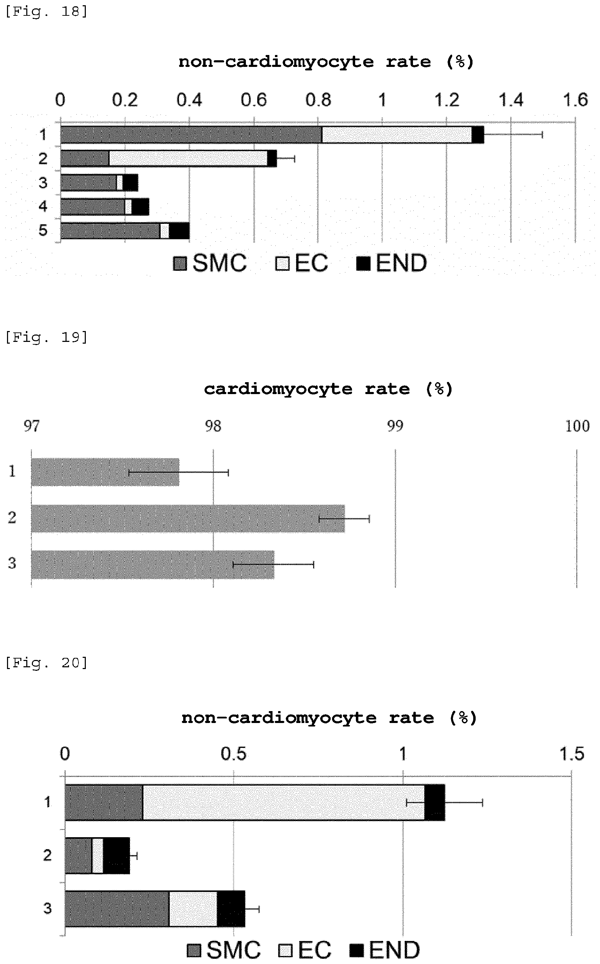

   Fig. 18 shows non-cardiomyocyte rate after treatment with compound (experiment number 1 is mean of n=4, experiment number 2 is mean of n=3 (standard deviation), experiment number 3 is mean of n=2, n=1 in experiment numbers 4, 5) (END: endoderm lineage cell, SMC: smooth muscle-like cell, EC: endothelial-like cell). The vertical axis shows experiment number, and the horizontal axis shows non-cardiomyocyte rate.
[Fig. 19]
   Fig. 19 shows mean cardiomyocyte rate after treatment with compound (standard deviation) (n=4; n=3 in experiment number 2). The vertical axis shows experiment number, and the horizontal axis shows cardiomyocyte rate.
[Fig. 20]
   Fig. 20 shows mean non-cardiomyocyte rate after treatment with compound (standard deviation) (n=4) (END: endoderm lineage cell, SMC: smooth muscle-like cell, EC: endothelial-like cell). The vertical axis shows experiment number, and the horizontal axis shows non-cardiomyocyte rate.
[Fig. 21]

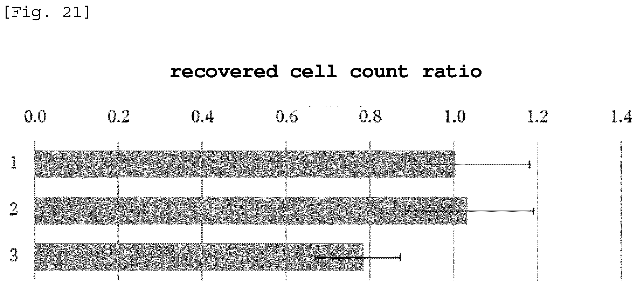

   Fig. 21 shows mean relative ratio of recovered cell count after treatment with compound wherein the recovered cell count without treatment with compound is 1 (standard deviation) (n=4). The vertical axis shows experiment number, and the horizontal axis shows relative ratio of recovered cell count.

### (Detailed Description of the Invention)

### 1. Production method of cell population containing cardiomyocytes

The present invention provides a method for producing a cell population containing cardiomyocytes (hereinafter also to be referred to as "the production method of the present invention"). The production method of the present invention includes (1) a step of bringing a receptor-type tyrosine kinase inhibitor into contact with a cell population containing cardiomyocytes or cardiac progenitor cells, and other cells, and (2) a step of culturing the cell population. In the production method of the present invention, EGF receptor inhibitors are excluded from the "receptor-type tyrosine kinase inhibitor" in the above-mentioned (1).

In the present specification, "cardiomyocytes" means a cell positive for at least one of sarcomeric α-actinin, cardiac troponin T (cTnT), and troponin I type 1 (TNNI1), and is preferably a sarcomeric α-actinin-positive cell. Typically, it is a cardiac muscle cell having self-beating ability. The "cardiac progenitor cell" means a progenitor cell for the aforementioned cardiomyocytes, and positive for at least one of Nkx2.5, GATA4, MEF2C, and MESP1. The above-mentioned "other cell" means a cell that is not cardiomyocyte or cardiac progenitor cell (hereinafter also to be referred to as "non-cardiomyocyte"), and examples of the specific cell include smooth muscle cell, endothelial cell, stem cell (e.g., pluripotent stem cell), and the like.

In the present specification, "positive" means that a protein or a gene is expressed in an amount detectable by at least any method known in the art. Protein can be detected by an immunological assay using an antibody, such as ELISA, immunostaining, or flow cytometry. In the case of a protein that is intracellularly expressed and does not appear on the cell surface (e.g., transcription factor or subunit thereof, and the like), a reporter protein is expressed together with the protein, and the target protein can be detected by detecting the reporter protein. Gene can be detected by, for example, a nucleic acid amplification method and/or a nucleic acid detection method such as RT-PCR, biochip (e.g., microarray), RNA seq, or the like. The expression of a protein or a gene can be determined by a general method. For example, when flow cytometry is utilized and the expression level is relatively high as compared with the expression level of the control group showing negative protein expression, the protein can be determined to be detectably expressed.

In the present specification, "negative" means that the expression level of the protein or a gene is less than the lower detection limit by all or any of the above-mentioned known methods. The lower detection limit of the expression of a protein or a gene may vary depending on each method, but can be determined by a general method.

In the present specification, the "cell population" means a population composed of two or more cells of the same type or different types. The "cell population" also means a mass of cells of the same type or different types. A cell population containing cardiomyocytes or cardiac progenitor cells, and non-cardiomyocytes to be used in the aforementioned step (1) can be produced by culturing pluripotent stem cells in a medium for cardiomyocyte differentiation. Therefore, the production method of the present invention may include, before the aforementioned step (1), step (0) of inducing differentiation of cardiomyocytes or cardiac progenitor cells from pluripotent stem cells.

Examples of the pluripotent stem cell to be used in the present invention include induced pluripotent stem cell (iPS cell), embryonic stem cell (ES cell), embryonic stem cell derived from clone embryo obtained by nucleus transplantation (nuclear transfer Embryonic stem cell: ntES cell), multipotent germline stem cell (mGS cell), embryonic germ cell (EG cell), Muse cell (multi-lineage differentiating stress enduring cell). iPS cell is preferred (human iPS cell is more preferred). When the above-mentioned pluripotent stem cell is ES cell or any cell derived from human embryo, the cell may be a cell produced by destroying the embryo or a cell prepared without destroying the embryo. Preferably, it is a cell prepared without destroying the embryo. The above-mentioned pluripotent stem cell is preferably derived from a mammal (e.g., mouse, rat, hamster, guinea pig, dog, monkey, orangutan, chimpanzee, human), more preferably human. Therefore, the pluripotent stem cell to be used in the present invention is most preferably human iPS cell.

The "induced pluripotent stem cell (iPS cell)" refers to a cell obtained by reprogramming by introducing a specific factor (nuclear reprogramming factor) into a mammalian somatic cell or undifferentiated stem cell. At present, there are various types of "induced pluripotent stem cells (iPS cell)". In addition to the iPS cell established by Yamanaka et al. by introducing four factors of Oct3/4, Sox2, Klf4, and c-Myc into mouse fibroblasts (Takahashi K, Yamanaka S., Cell, (2006) 126: 663-676), human cell-derived iPS cells established by introducing the same four factors into human fibroblasts (Takahashi K, Yamanaka S., et al. Cell, (2007) 131: 861-872.), Nanog-iPS cell established by selecting Nanog expression as an index after introducing the above-mentioned four factors (Okita, K., Ichisaka, T., and Yamanaka, S. (2007). Nature 448, 313-317.), iPS cell prepared by a method that does not contain c-Myc (Nakagawa M, Yamanaka S., et al. Nature Biotechnology, (2008) 26, 101-106), and iPS cell established by introducing six factors by a virus-free method (Okita K et al. Nat. Methods 2011 May; 8(5):409-12, Okita K et al. Stem Cells. 31(3):458-66.) can also be used. In addition, induced pluripotent stem cell established by introducing four factors of OCT3/4, SOX2, NANOG, and LIN28 produced by Thomson et al. (Yu J., Thomson JA. et al., Science (2007) 318: 1917-1920.), induced pluripotent stem cell produced by Daley et al. (Park IH, Daley GQ. et al., Nature (2007) 451: 141-146), induced pluripotent stem cell produced by Sakurada et al. (JP-A-2008-307007), and the like can also be used.

In addition, any of the induced pluripotent stem cells known in the art which are described in all published papers (e.g., Shi Y., Ding S., et al., Cell Stem Cell, (2008) Vol3, Issue 5, 568-574; , Kim JB., Scholer HR., et al., Nature, (2008) 454, 646-650; Huangfu D., Melton, DA., et al., Nature Biotechnology, (2008) 26, No 7, 795-797) or patents (e.g., JP-A-2008-307007, JP-A-2008-283972, US2008-2336610, US2009-047263, WO2007-069666, WO2008-118220, WO2008-124133, WO2008-151058, WO2009-006930, WO2009-006997, WO2009-007852) can be used. As the induced pluripotent stem cell line, various iPS cell lines established by NIH, RIKEN, Kyoto University, and the like can be used. For example, in the case of human iPS cell line, HiPS-RIKEN-1A strain, HiPS-RIKEN-2A strain, HiPS-RIKEN-12A strain, and Nips-B2 strain of RIKEN, and 253G1 strain, 201B7 strain, 409B2 strain, 454E2 strain, 606A1 strain, 610B1 strain, 648A1 strain, and iPS cell stock for regenerative medicine of Kyoto University, and the like can be mentioned.

The "somatic cell" in the present specification means any animal cell excluding germ line cells and differentiated totipotent cells such as ovum, oocyte, ES cells and the like (preferably, cells of mammals inclusive of human). Somatic cell is not particularly limited and encompasses any of somatic cells of fetuses, somatic cells of neonates, and mature healthy or pathogenic somatic cells, and any of primary cultured cells, passage cells, and established lines of cells. Specific examples of the somatic cell include (1) tissue stem cells (somatic stem cells) such as neural stem cell, hematopoietic stem cell, mesenchymal stem cell, dental pulp stem cell, and the like, (2) tissue progenitor cell, (3) differentiated cells such as lymphocyte, epithelial cell, endothelial cell, myocyte, fibroblast (skin cell, etc.), hair cell, hepatocyte, gastric mucosal cell, enterocyte, splenocyte, pancreatic cell (pancreatic exocrine cell, etc.), brain cell, lung cell, renal cell, adipocyte, and the like, and the like.

ES cell is a pluripotent and self-replicating stem cell established from the inner cell mass of early embryo (e.g., blastocyst) of a mammal such as human, mice, or the like. ES cell was discovered in mouse in 1981 (M.J. Evans and M.H. Kaufman (1981), Nature 292:154-156), after which ES cell line was also established in primates such as human, monkey and the like (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cell can be established by removing the inner cell mass from the blastocyst of a fertilized egg of the target animal and culturing the inner cell mass on a fibroblast feeder. Methods for establishing and maintaining human and monkey ES cells are described in, for example, USP5,843,780; Thomson JA, et al. (1995), Proc Natl. Acad. Sci. USA. 92:7844-7848; Thomson JA, et al. (1998), Science. 282:1145-1147; Suemori H. et al. (2006), Biochem. Biophys. Res. Commun., 345:926-932; Ueno M. et al. (2006), Proc. Natl. Acad. Sci. USA, 103:9554-9559; Suemori H. et al. (2001), Dev. Dyn., 222:273-279; Kawasaki H. et al. (2002), Proc. Natl. Acad. Sci. USA, 99:1580-1585; Klimanskaya I. et al. (2006), Nature. 444:481-485, and the like. Alternatively, ES cell can be established using only single blastomere of an embryo in the cleavage stage before the blastocyst stage (Chung Y. et al. (2008), Cell Stem Cell 2: 113-117), or can also be established using embryo that has stopped developing (Zhang X. et al. (2006), Stem Cells 24: 2669-2676.). As for "ES cell", various mouse ES cell lines established by inGenious targeting laboratory, RIKEN, and the like can be used for mouse ES cells. For human ES cell, various human ES cell lines established by the University of Wisconsin, NIH, RIKEN, Kyoto University, the National Center for Child Health and Development, Cellartis, and the like can be used. For example, as human ES cell lines, CHB-1 to CHB-12 strains, RUES1 strain, RUES2 strain, HUES1 to HUES28 strains, and the like distributed by ESI Bio, and H1 strain, H9 strain, and the like distributed by WiCell Research, and KhES-1 strain, KhES-2 strain, KhES-3 strain, KhES-4 strain, KhES-5 strain, SSES1 strain, SSES2 strain, SSES3 strain, and the like distributed by RIKEN can be utilized.
nt ES cell (nuclear transfer ES cell) is an ES cell derived from a cloned embryo prepared by a nuclear transplantation technique, and has almost the same property as the ES cell derived from a fertilized egg (Wakayama T. et al. (2001), Science, 292: 740-743; S. Wakayama et al. (2005), Biol. Reprod., 72: 932-936; J. Byrne et al. (2007), Nature, 450: 497-502). That is, an ES cell established from an inner cell mass of a blastocyst derived from a cloned embryo obtained by substituting the nucleus of an unfertilized egg with the nucleus of a somatic cell is an nt ES (nuclear transfer ES) cell. For production of an nt ES cell, a combination of the nuclear transplantation technique (Cibelli J.B. et al. (1998), Nature Biotechnol., 16: 642-646) and the ES cell production technique (mentioned above) is used (Kiyoka Wakayama et al., (2008), Experimental Medicine, Vol. 26, No. 5 (Suppl.), pp. 47 to 52). In nuclear transplantation, reprogramming can be performed by injecting the nucleus of a somatic cell to an enucleated unfertilized egg of a mammal, and culturing for a few hours.
mGS cell is a pluripotent stem cell derived from the testis, which becomes the origin for spermatogenesis. This cell can be differentiation induced into various lines of cells, like ES cells and shows properties of, for example, generation of a chimeric mouse by transplantation into a mouse blastocyst and the like (Kanatsu-Shinohara M. et al. (2003) Biol. Reprod., 69: 612-616; Shinohara K. et al. (2004), Cell, 119: 1001-1012). It is self-renewable in a culture medium containing a glial cell line-derived neurotrophic factor (GDNF), can produce a germ stem cell by repeating passages under culture conditions similar to those for ES cells (Masanori Takehashi et al., (2008), Experimental Medicine, Vol. 26, No. 5 (Suppl.), pp. 41 to 46, YODOSHA (Tokyo, Japan)).

EG cell is a cell having pluripotency similar to that of ES cells, which is established from a primordial germ cell at the prenatal period. It can be established by culturing a primordial germ cell in the presence of a substance such as LIF, bFGF, a stem cell factor, or the like (Matsui Y. et al. (1992), Cell, 70: 841-847; J.L. Resnick et al. (1992), Nature, 359: 550-551).

Muse cell is an in vivo endogeneous non-neoplastic pluripotent stem cell and can be produced, for example, by the method described in WO 2011/007900. In more detail, Muse cell is a cell having pluripotency, which is obtained by subjecting a fibroblast or a bone marrow stromal cell to a trypsin treatment for a long time, preferably for 8 hr or for 16 hr, and thereafter culturing the cells in a suspended state, and positive for SSEA-3 and CD105.

The aforementioned step (0) is not particularly limited as long as pluripotent stem cells can be induced to differentiate into cardiomyocytes or cardiac progenitor cells. For example, differentiation into cardiomyocytes or cardiac progenitor cells can be induced by culturing pluripotent stem cells in a medium for cardiomyocyte differentiation. In one embodiment of the present invention, the aforementioned step (0) may include (0-1) step of inducing differentiation of pluripotent stem cells into mesodermal cells, and (0-2) step of inducing differentiation of the mesodermal cells into cardiomyocytes or cardiac progenitor cells. In the present specification, the "medium for cardiomyocyte differentiation" means a medium containing a factor that promotes induction of differentiation into cardiomyocytes such as cytokine and the like (hereinafter sometimes referred to as "cardiomyocyte differentiation-inducing factor") and a basal medium. The above-mentioned factor that promotes induction of differentiation into cardiomyocytes encompasses a factor necessary for inducing differentiation into intermediate cells (e.g., mesodermal cell and the like) in the differentiation induction process from pluripotent stem cells into cardiomyocytes or cardiac progenitor cells.

Examples of the basal medium to be used in the present invention include StemFit (e.g., StemFit AK03N, StemFit AK02N) (Ajinomoto Co., Inc.), StemPro-34 (Thermo Fisher Scientific), PECM (Primate ES Cell medium), GMEM (Glasgow Minimum Essential medium), IMDM (Iscove's Modified Dulbecco's medium), 199 medium, Eagle's Minimum Essential medium (EMEM), αMEM, Dulbecco's modified Eagle's medium (DMEM), Ham's F12 medium, RPMI 1640 medium, Fischer's medium, and a combined medium thereof, and the like.

It is possible to appropriately add ROCK inhibitors (e.g., Y-27632, Fasudil/HA1077, SR3677, GSK269962, H-1152, Wf-536, etc.), serums (e.g., bovine embryo serum (FBS), human serum, horse serum, etc.) or serum substitutes, insulins, various vitamins (e.g., vitamin C (e.g., ascorbic acid)), various amino acids such as L-glutamine, non-essential amino acid, and the like, 2-mercaptoethanol, thioglycerol (e.g., α-monothioglycerol (MTG)), various cytokines, stem cell factors (SCF (Stem cell factor)), activin, etc.), various hormones, various growth factors (leukemia inhibitory factor (LIF), basic fibroblast growth factor (bFGF), TGF-β, etc.), various extracellular matrixs, various cell adhesion molecules, antibiotics such as penicillin/streptomycin, puromycin, and the like, pH indicators such as phenol red and the like, and the like to the basal medium. Examples of the serum substitute include albumin, transferrin, fatty acid, insulin, collagen precursor, trace element, Knockout Serum Replacement (KSR), ITS-supplement, and mixtures thereof, and the like.

In the present invention, the "Vitamin Cs" means L-ascorbic acid and derivatives thereof, and "L-ascorbic acid derivative" means derivatives that become vitamin C by enzymatic reaction in the living body. Examples of the derivatives of L-ascorbic acid to be used in the present invention include vitamin C phosphate (e.g., ascorbic acid 2-phosphate), ascorbic acid glucoside, ascorbyl ethyl, vitamin C ester, ascorbyl tetrahexyldecanoate, ascorbyl stearate, and ascorbyl 2-phosphate 6-palmitate. Preferred is vitamin C phosphate (e.g., ascorbic acid 2-phosphate). Examples of the vitamin C phosphate include salts of L-ascorbic acid phosphate such as L-ascorbic acid phosphate Na and L-ascorbic acid phosphate Mg.

The induced pluripotent stem cells or embryoid bodies may be cultured by adherent culture or suspension culture. In cases of adherent culture, the culturing may be carried out in a culture vessel coated with an extracellular matrix component, and/or may be co-cultured with feeder cells. While the feeder cell is not particularly limited, for example, fibroblast (mouse embryo fibroblast (MEF), mouse fibroblast (STO), and the like) can be mentioned. Feeder cells are preferably inactivated by a method known per se, for example, radiation (gamma-ray and the like) irradiation, treatment with anticancer agent (mitomycin C and the like), or the like. As the extracellular matrix component, fibrous proteins such as Matrigel (Niwa A, et al., PLoS One.6(7): e22261, 2011), gelatin, collagen, elastin, and the like, glucosaminoglycan and proteoglycan such as hyaluronic acid, chondroitin sulfate, and the like, cell adhesion proteins such as fibronectin, vitronectin, laminin, and the like, and the like can be mentioned.

The culture temperature conditions are not particularly limited. The temperature is, for example, about 37°C to 42°C, preferably about 37°C to 39°C. The culture may be carried out under low-oxygen conditions, and the low-oxygen condition in the present invention means, for example, oxygen concentration of 15%, 10%, 9%, 8%, 7%, 6%, 5% or lower than these.

Suspension culture means culturing cells in a state of being non-adhesive to the culture container, and is not particularly limited. It can be performed using a culture container free of an artificial treatment (e.g., coating treatment with extracellular matrix or the like) in order to improve adhesiveness to cells, or a culture container artificially treated to suppress adhesion (e.g., coating treatment with poly-hydroxyethyl methacrylic acid (poly-HEMA) or non-ionic surface-active activity polyol (Pluronic F-127, etc.)). For example, suspension culture may be performed using an incubator provided with an impeller, such as single-use bioreactor (Biott Corporation), single-use bioreactor (Thermo Fisher), single-use bioreactor (Sartorius Stedim), single-use bioreactor (GE Healthcare Life Sciences), or the like. The kind and the stirring rate of the incubator to be used can be appropriately selected by those of ordinary skill in the art according to the type of cells to be cultured. For example, the stirring rate may be, though not limited to, 0 to 100 rpm, 20 to 80 rpm, or 45 to 65rpm.

During the suspension culture, it is preferable to form an embryoid body (EB) and culture the same. Therefore, the aforementioned step (0) may include a step of forming an embryoid body from pluripotent stem cells. In such step, it is preferable to dissociate colony-forming pluripotent stem cells into single cells and then form the embryoid body. In the step of dissociating pluripotent stem cells, the cells forming a population by adhering to each other are dissociated (separated) into individual cells. The method for dissociating pluripotent stem cells includes, for example, a method of mechanical dissociation, and a dissociation method using a dissociation solution having protease activity and collagenase activity (e.g., Accutase^{™}, Accumax^{™}, and the like) or a dissociation solution having collagenase activity alone. Preferably, a method of dissociating pluripotent stem cells by using a dissociation solution having protease activity and collagenase activity (particularly preferably Accumax^{™}) is used. The medium used in the above-mentioned step preferably contains thioglycerol, L-glutamine and/or ascorbic acid.

Cardiomyocyte differentiation-inducing factors used in the above-mentioned step (0-1) include, for example, Wnt signal activating substance, activin A, BMP4, and bFGF, and these may be used alone or in combination. In one embodiment of the present invention, activin A, BMP4, and bFGF are used in combination. In addition, the medium used in the above-mentioned step (0-1) preferably contains thioglycerol, L-glutamine and/or ascorbic acid.

In the present specification, the "Wnt signal activator" means a substance that activates the Wnt signal pathway. Examples of the Wnt signal activator include Wnt protein, GSK3β inhibitor (e.g., BIO, CHIR99021, etc.), and the like. These may be used alone or in combination. When a Wnt signal activator is used, the concentration thereof in the medium is not particularly limited. When BIO or CHIR99021 is used as the Wnt signal activator, it is preferably used at a final concentration of 100 nM to 100 µM, preferably 1 µM to 10 µM, in the medium.

When activin A is used, the concentration thereof in the medium is preferably 1 ng/ml to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

When BMP4 is used, the concentration thereof in the medium is preferably 1 ng/ml to 1 µg/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, 100 ng/ml, 200 ng/ml, 300 ng/ml, 400 ng/ml, 500 ng/ml, 600 ng/ml, 700 ng/ml, 800 ng/ml, 900 ng/ml, or 1 µg/ml.

When bFGF is used, the concentration thereof in the medium is preferably 1 ng/ml to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

The period of the above-mentioned step (0-1) is not particularly limited as long as mesodermal cells are obtained. It is preferably not less than 12 hr (e.g., 1 day, 2 days, or longer), or may be not more than 6 days (e.g., 5 days, 4 days, 3 days, or shorter). In addition, whether or not mesodermal cells are obtained may be monitored, and in this case, it can be determined by the expression of the mesoderm marker gene. Examples of the mesoderm marker gene include T, MIXL1, NODAL, and the like.

Examples of the cardiomyocyte differentiation-inducing factor used in the above-mentioned step (0-2) include Wnt inhibitor and VEGF. These may be used alone or in combination. The medium used in the above-mentioned step (0-2) preferably contains thioglycerol, L-glutamine and/or ascorbic acid.

In the present specification, the "Wnt inhibitor" means a substance that inhibits signal transduction induced by the binding of Wnt to receptors subjected to the accumulation of β-catenin. It may be a substance that inhibits the binding to the Frizzled family of receptors or a substance that promotes the degradation of β-catenin. Examples of the Wnt inhibitor include DKK1 protein (e.g., in the case of human, NCBI accession number: NM_012242), sclerostin (e.g., in the case of human, NCBI accession number: NM_025237), IWR-1 (Merck Millipore), IWP-2 (Sigma-Aldrich), IWP-3 (Sigma-Aldrich), IWP-4 (Sigma-Aldrich), PNU-74654 (Sigma-Aldrich), XAV939 (Sigma-Aldrich), derivatives thereof, and the like. Among these, IWP-3 or IWP-4 is preferred. Only one kind of Wnt inhibitor may be used or plural kinds thereof may be used in combination.

When a Wnt inhibitor is used, its concentration in the medium is preferably 1 nM to 50 µM, and is, for example, 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though not limited to these. It is more preferably 1 µM.

When VEGF is used, its concentration in the medium is preferably 1 to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

In the above-mentioned step (0-2), a BMP inhibitor and/or a TGFβ inhibitor may be further added as a cardiomyocyte differentiation-inducing factor to the basal medium. In the present specification, examples of the "BMP inhibitor" include proteinaceous inhibitors such as Chordin, Noggin, Follistatin, and the like, Dorsomorphin (6-[4-(2-piperidin-1-yl-ethoxy)phenyl]-3-pyridin-4-yl-pyrazolo[1,5-a]pyrimidine) and a derivative thereof (P. B. Yu et al. (2007), Circulation, 116: II_60; P. B. Yu et al. (2008), Nat. Chem. Biol., 4: 33-41; J. Hao et al. (2008), PLoS ONE, 3(8): e2904), LDN-193189 (4-(6-(4-(piperazin-1-yl)phenyl)pyrazolo[1,5-a]pyrimidin-3-yl)quinoline), and the like. Among these, Dorsomorphin is preferred. Only one kind of BMP inhibitor or TGFβ inhibitor may be used or plural kinds thereof may be used in combination.

When a BMP inhibitor is used, its concentration in the medium is preferably 1 nM to 50 µM, and is, for example, 1 nM, 10 nM, 50 nM, 100 nM, 500 nM, 600 nM, 700 nM, 800 nM, 900 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though not limited to these.

In the present specification, the TGFβ inhibitor means a substance that inhibits signal transduction from the binding of TGFβ to a receptor, leading to SMAD. It may be a substance that inhibits the binding to ALK family of receptors, or a substance that inhibits phosphorylation of SMAD by ALK family. Examples of the TGFβ inhibitor include Lefty-1 (for example, mouse: NM_010094, human: NM_020997 in NCBI Accession No.), SB431542, SB202190 (all R.K. Lindemann et al., Mol. Cancer, 2003, 2: 20), SB505124 (GlaxoSmithKline), NPC30345, SD093, SD908, SD208 (Scios), LY2109761, LY364947, LY580276 (Lilly Research Laboratories), A-83-01 (WO 2009146408), derivatives thereof, and the like. Among them, SB431542 is preferred.

When a TGFβ inhibitor is used, its concentration in the medium is preferably 1 nM to 50 µM, and is, for example, 1 nM, 10 nM, 50 nM, 100 nM, 500 nM, 750 nM, 1 µM, 2 µM, 3 µM, 4 µM, 5 µM, 5.2 µM, 5.4 µM, 5.6 µM, 5.8 µM, 6 µM, 7 µM, 8 µM, 9 µM, 10 µM, 15 µM, 20 µM, 25 µM, 30 µM, 40 µM, or 50 µM, though not limited to these.

The period of the above-mentioned step (0-2) is not particularly limited as long as cardiomyocytes or cardiac progenitor cells are obtained. It is, for example, not less than 1 day (e.g., 1 day, 2 days, 3 days, 4 days, 5 days, 6 days, 7 days, or longer). Since long-term culture does not affect the establishment of cardiomyocytes or cardiac progenitor cells, the upper limit is not particular set, but it is typically not more than 40 days. In addition, whether or not cardiomyocytes or cardiac progenitor cells are obtained may be monitored. In this case, it can be confirmed by the number of beating cardiomyocytes, expression of the markers of cardiomyocytes or cardiac progenitor cells, expression of ion channels, response to electric physiological stimulation, or the like.

The aforementioned step (0) may further contain step (0-3) of culturing cardiomyocytes or cardiac progenitor cells, which are obtained in step (0-2), in the presence or absence of VEGF and/or bFGF. The medium used in this step preferably contains thioglycerol, L-glutamine and/or ascorbic acid. In addition, the medium used in this step may also contain a compound for myocardial maturation (e.g., N-(1,1-dioxo-2,3-dihydro-1H-1-benzothiophen-5-yl)-2-(4-{5-[1-oxo-5-(piperidin-1-yl)-1,3-dihydro-2H-isoindol-2-yl]-1H-benzoimidazol-2-yl}phenoxy)acetamide) and/or a multikinase inhibitor (e.g., 2-(4-{3-[3-(2-amino-2-phenylethoxy)-4-cyanophenyl]pyrazolo[1,5-a]pyrimidin-6-yl}-1H-pyrazol-1-yl)-N-(2-methoxyethyl)acetamide) .

When VEGF is used in step (0-3), its concentration in the medium is preferably 1 to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

When bFGF is used in step (0-3), its concentration in the medium is preferably 1 to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml. It is more preferably 5 ng/ml.

The period of the above-mentioned step (0-3) is not particularly limited. For example, it is not less than one day (e.g., 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, or longer). Since long-term culture does not affect the establishment of cardiomyocytes or cardiac progenitor cells, the upper limit is not particular set, but it is typically not more than 60 days. The above-mentioned step (0-3) may improve efficiency of differentiation into cardiomyocytes or cardiac progenitor cells.

In addition, embryoid bodies may be dissociated by a method similar to that described above, prior to the above-mentioned step (0-2) or step (0-3).

The method specifically described above is merely an example, and the method is not limited to the above-mentioned method. For example, a method of coculturing END2 cells, which are mouse-derived supporting cells, and pluripotent stem cells (Mummery, C., et al., Differentiation of human embryonic stem cells to cardiomyocytes: role of coculture with visceral endoderm-like cells. Circulation. 107(21), 2733-40 (2003)), a method of inducing cardiomyocytes by culturing embryoid bodies with BMP4, FGF2, insulin, and serum (Paul, W B., et al., A Universal System for Highly Efficient Cardiac Differentiation of Human Induced Pluripotent Stem Cells That Eliminates Interline Variability. PLoSone. 6(4), e18293 (2011)), and the like can be mentioned. In addition, a method of inducing differentiation of cardiomyocytes without using cytokine in adhesion culture (Lian X, et al., Robust cardiomyocyte differentiation from human pluripotent stem cells via temporal modulation of canonical Wnt signaling., Proc Natl Acad Sci USA., 2012 July 3; 109(27): E1848-57), a method of inducing differentiation of cardiomyocytes by adhesion culture and suspension culture in combination without using cytokine (Minami I, et al., A small molecule that promotes cardiac differentiation of human pluripotent stem cells under defined, cytokine-and xeno-free conditions., Cell Rep., 2012 Nov 29; 2(5): 1448-60), and the like may also be used.

A cell population with high purity of cardiomyocytes, compared with the cell population before contact with a receptor-type tyrosine kinase inhibitor, can be produced by bringing a cell population containing cardiomyocytes or cardiac progenitor cells obtained as mentioned above into contact with the receptor-type tyrosine kinase inhibitor, and then culturing the cell population. That is, cardiomyocytes can be purified using a receptor-type tyrosine kinase inhibitor. Therefore, in another embodiment of the present invention, a method for purifying cardiomyocytes, including (1') a step of bringing a receptor-type tyrosine kinase inhibitor into contact with a cell population containing cardiomyocytes or cardiac progenitor cells, and non-cardiomyocytes, the cell population being obtained by culturing pluripotent stem cells in a medium for cardiomyocyte differentiation, and (2') a step of culturing the cell population (hereinafter also to be referred to as "the purification method of the present invention") is provided.

In the present specification, purifying cardiomyocytes means that the proportion of cardiomyocytes in a cell population (number of cardiomyocytes in cell population/total number of cells in cell population) increases because the rate of decrease in the cell count of non-cardiomyocytes ("cell count" means viable cell count, hereinafter the same) exceeds the rate of decrease in cardiomyocytes, or the proliferation of non-cardiomyocytes is inhibited and thus the proliferation rate of cardiomyocytes exceeds, each due to a receptor-type tyrosine kinase inhibitor. Therefore, it is distinguished from an increase in the proportion of cardiomyocytes due to the promotion of differentiation induction from cardiac progenitor cells into cardiomyocytes or inhibition of differentiation induction from cardiac progenitor cells into cells other than cardiomyocytes. Without wishing to be bound by any theory, it is assumed that the decrease in the cell count due to a receptor-type tyrosine kinase inhibitor is the result of the induction of cell apoptosis by the receptor-type tyrosine kinase inhibitor.

In the aforementioned steps (1) and (1'), the period of contact of a cell population containing cardiomyocytes or cardiac progenitor cells and a receptor-type tyrosine kinase inhibitor is not particularly limited. For example, it is preferably not less than 1 hr (e.g., 2 hr, 3 hr, 5 hr, 12 hr, 1 day, 2 days, 3 days, or longer). Since long-term culture does not affect the establishment of cardiomyocytes or cardiac progenitor cells, the upper limit is not particular set, but it is typically not more than 60 days (e.g., 50 days, 40 days, 30 days, 20 days, 14 days, 13 days, 12 days, 11 days, or shorter). The contact between a cell population containing cardiomyocytes or cardiac progenitor cells and a receptor-type tyrosine kinase inhibitor may be performed by adding a receptor-type tyrosine kinase inhibitor to a medium containing the cell population, or by seeding the cell population in a medium previously supplemented with a receptor-type tyrosine kinase inhibitor. The timing of contacting the receptor-type tyrosine kinase inhibitor is not particularly limited as long as the cell population contains cardiomyocytes or cardiac progenitor cells. For example, contacting in the above-mentioned step (0-2) or (0-3) is preferred. Based on the start date of induction of differentiation of pluripotent stem cells, contacting on or after 4 days (e.g., 5 days, 6 days, 7 days, or later) from the start of differentiation induction is preferred.

Examples of the receptor-type tyrosine kinase to be inhibited by the receptor-type tyrosine kinase inhibitor used in the present invention include EGF receptors (also to be referred to as ErbB or HER) (e.g., ErbB1 (EGFR), ErbB2 (HER2), ErbB3 (HER3), ErbB4 (HER4)), insulin receptors (e.g., IR-A, IR-B), insulin-like growth factor 1 receptor, VEGF receptors (e.g., VEGFR-1 (Flt-1), VEGFR-2 (KDR/Flk-1), VEGFR-3 (Flt-4)), PDGF receptors (e.g., PDGFRα, PDGFRβ), HGF receptors (also to be referred to as c-Met), FGF receptors (e.g., FGFR1, FGFR2, FGFR3, FGFR4), CCK, NGF receptors (also to be referred to as Trk receptor) (e.g., TrkA, TrkB, TrkC), Eph (Ephrin) receptors (e.g., EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6), AXL (also to be referred to as TAM receptor), TIE (e.g., TIE-1, TIE-2), RYK, DDR (e.g., DDR1), RET, ROS, LTK, ROR (e.g., ROR1, ROR2), MuSK, and LMR. Among them, VEGF receptors, PDGF receptors, HGF receptors, and FGF receptors are preferred as the targets of the receptor-type tyrosine kinase inhibitor. EGF receptor inhibitors are excluded from the receptor-type tyrosine kinase inhibitor used in the aforementioned step (1); however, EGF receptor inhibitors can be used as the receptor-type tyrosine kinase inhibitor used in the aforementioned step (1').

In the present specification, the receptor-type tyrosine kinase inhibitor may have other activities, such as inhibitory activity against other receptor-type tyrosine kinases, or may have inhibitory activity specific to one type of receptor-type tyrosine kinase, as long as it has inhibitory activity against at least one of the above-mentioned receptor-type tyrosine kinases. In addition, the receptor-type tyrosine kinase inhibitor other than EGF receptor inhibitors does not exclude those having inhibitory activity against receptor-type tyrosine kinases other than EGF receptors and having inhibitory activity against EGF receptors. In the present specification, the "EGF receptor inhibitor" refers to a substance having EGF receptor inhibitory activity superior to other activities, and specifically refers to a substance that inhibits at least 90% of EGF receptor or shows a 50% inhibitory concentration of the EGF receptor of not more than 10 µM.

Examples of the inhibitor to EGF receptor to be used in the present invention include Cetuximab, Erlotinib HCl (OSI-744), Gefitinib (ZD1839), Lapatinib (GW-572016) Ditosylate, Afatinib (BIBW2992), Canertinib (CI-1033), TAS6417, PD153035, MTX-211, HS-10296, Theliatinib (HMPL-309), Lapatinib, AG-490 (Tyrphostin B42), CP-724714, Dacomitinib (PF-00299804), WZ4002, Sapitinib (AZD8931), CUDC-101, AG-1478 (Tyrphostin AG-1478), PD153035 HCl, Pelitinib (EKB-569), AC480 (BMS-599626), AEE788 (NVP-AEE788), AP26113-analog (ALK-IN-1), OSI-420, WZ3146, HER2-Inhibitor-1, WZ8040, AST-1306, Rociletinib (CO-1686), Genistein, Varlitinib, Icotinib, TAK-285, WHI-P154, Daphnetin, PD168393, Tyrphostin 9, CNX-2006, AG-18, AZ5104, Lazertinib, AZD9291, CL-387785 (EKI-785), Olmutinib (BI 1482694), (-)-Epigallocatechin Gallate, Erlotinib, Gefitinib hydrochloride, Afatinib (BIBW2992) Dimaleate, AZD3759, Poziotinib (HM781-36B), Brigatinib (AP26113), Osimertinib mesylate, Naquotinib (ASP8273), Chrysophanic Acid, Nazartinib (EGF816), Norcantharidin, Lifirafenib (BGB-283), Lidocaine hydrochloride, Butein, EAI045, Cyasterone, Avitinib (AC0010), and the like.

Examples of the inhibitor to insulin receptor or insulin-like growth factor 1 receptor include HNMPA, GSK1904529A, Dovitinib (TKI-258) Dilactic Acid, Dovitinib (TKI258) Lactate, NVP-AEW541, Dovitinib (TKI-258), NVP-ADW742, Linsitinib (OSI-906), GSK1904529A, BMS-754807, TAE226 (NVP-TAE226), Ceritinib (LDK378), and the like.

Examples of the inhibitor to PDGFRα include N-[5-({2-[(cyclopropanecarbonyl)amino]imidazo[1,2-b]pyridazin-6-yl}oxy)-2-methylphenyl]-1,3-dimethyl-1H-pyrazole-5-carboxamide (compound of the formula (I))

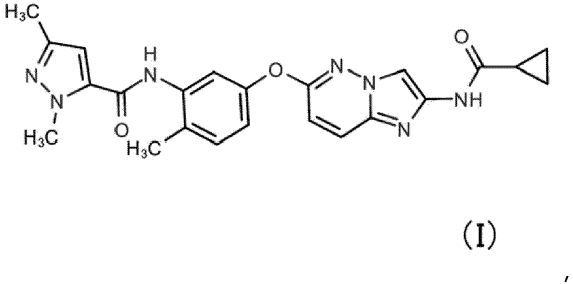

Ponatinib (AP24534), Telatinib, Amuvatinib (MP-470), Ki8751, KRN 633, Crenolanib (CP-868596), Axitinib, CP-673451, Tivozanib (AV-951), Nintedanib (BIBF 1120), Dovitinib (TKI-258, CHIR-258), Dovitinib (TKI258) Lactate, Dovitinib (TKI-258) Dilactic Acid, Masitinib (AB1010), and the like. Examples of the inhibitor to PDGFRβ include N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, CP-673451, Sunitinib Malate, Sunitinib, TSU-68 (SU6668, Orantinib), MK-2461, Sorafenib Tosylate, Linifanib (ABT-869), Axitinib, Crenolanib (CP-868596), Dovitinib (TKI-258) Dilactic Acid, Dovitinib (TKI-258, CHIR-258), Dovitinib (TKI258) Lactate, Tivozanib (AV-951), Nintedanib (BIBF 1120), Masitinib (AB1010), KRN 633, and the like.

Examples of the inhibitor to VEGFR-1 include N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, ZM 306416, Axitinib, Motesanib Diphosphate (AMG-706), Linifanib (ABT-869), MGCD-265, Cediranib (AZD2171), Foretinib (GSK1363089), OSI-930, Dovitinib (TKI-258, CHIR-258), Pazopanib HCl (GW786034 HCl), Pazopanib, Dovitinib (TKI-258) Dilactic Acid, Dovitinib (TKI258) Lactate, Cabozantinib (XL184, BMS-907351), Cabozantinib malate (XL184), Regorafenib (BAY 73-4506), Lenvatinib (E7080), Tivozanib (AV-951), Nintedanib (BIBF 1120), AEE788 (NVP-AEE788), Vatalanib (PTK787) 2HCl, KRN 633, Brivanib (BMS-540215), Brivanib Alaninate (BMS-582664), and the like. Examples of the inhibitor to VEGFR-2 include N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, N-{4-[(6,7-dimethoxyquinolin-4-yl)oxy]-3-fluorophenyl}-N'-(4-fluorophenyl)cyclopropane-1,1-dicarboxamide (compound of the formula (II))

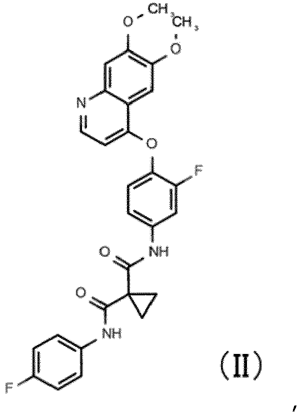

Cabozantinib (XL184, BMS-907351), Cabozantinib malate (XL184), Ki8751, Apatinib, Ponatinib (AP24534), ZM323881, LY2874455, Sorafenib Tosylate, BMS-794833, Golvatinib (E7050), RAF265 (CHIR-265), SKLB1002, Vandetanib (ZD6474), CYC116, Sunitinib Malate, Sunitinib, PD173074, Semaxanib (SU5416), TSU-68 (SU6668, Orantinib), Axitinib, Cediranib (AZD2171), Foretinib (GSK1363089), MGCD-265, Motesanib Diphosphate (AMG-706), Linifanib (ABT-869), Lenvatinib (E7080), Regorafenib (BAY 73-4506), Telatinib, Tivozanib (AV-951), OSI-930, Dovitinib (TKI-258) Dilactic Acid, Nintedanib (BIBF 1120), Dovitinib (TKI258) Lactate, Dovitinib (TKI-258, CHIR-258), Brivanib Alaninate (BMS-582664), Brivanib (BMS-540215), Pazopanib, Pazopanib HCl (GW786034 HCl), Vatalanib (PTK787) 2HCl, ENMD-2076 L-(+)-Tartaric acid, ENMD-2076 L-(+)-Tartaric acid, AEE788 (NVP-AEE788), KRN 633, and the like. Examples of the inhibitor to VEGFR-3 include N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, SAR131675, Axitinib, Foretinib (GSK1363089), Cediranib (AZD2171), Telatinib, MGCD-265, Lenvatinib (E7080), Cabozantinib (XL184, BMS-907351), Cabozantinib malate (XL184), Motesanib Diphosphate (AMG-706), Dovitinib (TKI-258, CHIR-258), Dovitinib (TKI-258) Dilactic Acid, Dovitinib (TKI258) Lactate, Nintedanib (BIBF 1120), Tivozanib (AV-951), ENMD-2076 L-(+)-Tartaric acid, ENMD-2076, Regorafenib (BAY 73-4506), Pazopanib HCl (GW786034 HCl), Pazopanib, KRN 633, Linifanib (ABT-869), AEE788 (NVP-AEE788), Vatalanib (PTK787)2HCl, and the like.

Examples of the inhibitor to HGF receptor include N-{4-[(6,7-dimethoxyquinolin-4-yl)oxy]-3-fluorophenyl}-N'-(4-fluorophenyl)cyclopropane-1,1-dicarboxamide, Crizotinib (PF-02341066), Cabozantinib (BMS-907351), Foretinib (GSK1363089), PHA-665752, SU11274, JNJ-38877618 (OMO-1), Glumetinib (SCC244), Altiratinib, SAR125844, SGX-523, BMS-777607, Tivantinib (ARQ 197), JNJ-38877605, PF-04217903, MGCD-265 analog, Capmatinib (INCB28060), BMS-754807, BMS-794833, AMG-208, MK-2461, Golvatinib (E7050), AMG-458, NVP-BVU972, AMG 337, Merestinib (LY2801653), S49076, Pulsatilla saponin D, Norcantharidin, NPS-1034, Savolitinib (AZD6094), and the like.

Examples of the inhibitor to FGFR1 include ASP5878, Ponatinib (AP24534), PD173074, Danusertib (PHA-739358), Brivanib Alaninate (BMS-582664), Brivanib (BMS-540215), TSU-68 (SU6668, Orantinib), SSR128129E, AZD4547, BGJ398 (NVP-BGJ398), LY2874455, Dovitinib (TKI-258, CHIR-258), Dovitinib (TKI258) Lactate, Dovitinib (TKI-258) Dilactic Acid, CH5183284 (Debio-1347), MK-2461, Nintedanib (BIBF 1120), and the like. Examples of the inhibitor to FGFR2 include ASP5878, BGJ398 (NVP-BGJ398), AZD4547, LY2874455, CH5183284 (Debio-1347), Nintedanib (BIBF 1120), MK-2461, and the like. Examples of the inhibitor to FGFR3 include ASP5878, BGJ398 (NVP-BGJ398), AZD4547, LY2874455, Dovitinib (TKI-258) Dilactic Acid, Dovitinib (TKI258) Lactate, Dovitinib (TKI-258, CHIR-258), CH5183284 (Debio-1347), MK-2461, Nintedanib (BIBF 1120), and the like. Examples of the inhibitor to FGFR4 include ASP5878, LY2874455, AZD4547, CH5183284 (Debio-1347), Nintedanib (BIBF 1120), and the like.

Examples of the inhibitor to NGF receptor include BMS-754807, GW441756, DS-6051b, GNF-5837, CH7057288, Altiratinib, Selitrectinib (LOXO-195), BMS-935177, Entrectinib (RXDX-101), Sitravatinib (MGCD516), PF-06273340, Belizatinib (TSR-011), Larotrectinib (LOXO-101) sulfate, and the like.

Examples of the inhibitor to Eph include NVP-BHG712, Sitravatinib (MGCD516), and the like.

Examples of the inhibitor to AXL include BMS-777607, Bemcentinib (R428), Cabozantinib malate (XL184), UNC2250, Dubermatinib (TP-0903), UNC-2025, LDC1267, UNC2881, RXDX-106 (CEP-40783), S49076, Sitravatinib (MGCD516), 2-D08, Gilteritinib (ASP2215), NPS-1034, and the like.

Examples of the inhibitor to TIE include MGCD-265 analog, Tie2 kinase inhibitor, Altiratinib, Pexmetinib (ARRY-614), and the like.

In addition, other receptor-type tyrosine kinase inhibitors can be appropriately selected. As the receptor-type tyrosine kinase inhibitor, N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, N-{4-[(6,7-dimethoxyquinolin-4-yl)oxy]-3-fluorophenyl}-N'-(4-fluorophenyl)cyclopropane-1,1-dicarboxamide, AMG337, ASP5878, BGJ398, Foretinib, ZM323881, CP-673451, Crenolanib or Crizotinib is preferred. Only one kind of receptor-type tyrosine kinase inhibitor may be used, or plural kinds may be used in combination. To obtain cardiomyocytes with higher purity, other drugs (histone deacetylase inhibitor, etc.) may also be used simultaneously with the receptor-type tyrosine kinase inhibitor or in a step before or thereafter.

The above-mentioned inhibitor may contain one kind of the above-mentioned compound or a salt thereof, or may contain two or more kinds thereof.

The above-mentioned compound or a salt thereof can each be produced according to a method known per se.

When the above-mentioned compound is in the form of a salt, the salt is preferably a pharmacologically acceptable salt, and examples of such salt include a salt with an inorganic base, a salt with an organic base, a salt with an inorganic acid, a salt with an organic acid, a salt with a basic or acidic amino acid, and the like.

Preferable examples of the salt with an inorganic base include alkali metal salts such as sodium salt, potassium salt, and the like, alkaline earth metal salts such as calcium salt, magnesium salt, and the like, aluminum salt, ammonium salt, and the like.

Preferable examples of the salt with an organic base include a salt with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine, N,N-dibenzylethylenediamine, or the like.

Preferable examples of the salt with an inorganic acid include a salt with hydrochloric acid, hydrobromic acid, nitric acid, sulfuric acid, phosphoric acid, or the like.

Preferable examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, or the like.

Preferable examples of the salt with a basic amino acid include a salt with arginine, lysine, ornithine, or the like. Preferable examples of the salt with an acidic amino acid include a salt with aspartic acid, glutamic acid, or the like.

The above-mentioned compounds may be a hydrate, a non-hydrate, a solvate or a non-solvate.

Furthermore, the above-mentioned compounds may be labeled with or substituted by an isotope (e.g., ²H, ³H, ¹¹C, ¹⁴C, ¹⁸F, ³⁵S, ¹²⁵I, etc.) or the like.

Deuterium conversion forms wherein ¹H is converted to ²H (D) are also encompassed in the above-mentioned compounds.

Tautomers are also encompassed in the above-mentioned compounds.

The above-mentioned compounds may be a pharmaceutically acceptable cocrystal or cocrystal salt. The cocrystal or cocrystal salt means a crystalline substance constituted with two or more special solids at room temperature, each having different physical properties (e.g., structure, melting point, melting heat, hygroscopicity, solubility and stability). The cocrystal or cocrystal salt can be produced by a cocrystallization method known per se.

The concentration of the receptor-type tyrosine kinase inhibitor in the medium can be appropriately selected by those of ordinary skill in the art. For example, it is preferably 1 nM to 10 µM, particularly more preferably 10 nM to 3 µM, and is specifically 1 nM, 2 nM, 3 nM, 5 nM, 10 nM, 20 nM, 30 nM, 40 nM, 50 nM, 0.1 µM, 0.2 µM, 0.3 µM, 0.4 µM, 0.5 µM, 1.0 µM, 1.5 µM, 2 µM, 3 µM, 4 µM, 5 µM, 6 µM, 7 µM, 8 µM, 9 µM, or 10 µM. The concentration can also be changed as appropriate according to the kind of the compound. For example, when N-[5-({2-[(cyclopropanecarbonyl)amino]imidazo[1,2-b]pyridazin-6-yl}oxy)-2-methylphenyl]-1,3-dimethyl-1H-pyrazole-5-carboxamide is used, it is preferably 20 nM to 10 µM, when N-{4-[(6,7-dimethoxyquinolin-4-yl)oxy] -3-fluorophenyl}-N'-(4-fluorophenyl)cyclopropane-1,1-dicarboxamide is used, it is preferably 0.2 µM to 10 µM, when AMG337 is used, it is preferably 0.2 µM to 10 µM, when ASP5878 is used, it is preferably 1 nM to 1 µM, when BGJ398 is used, it is preferably 5 nM to 5 µM, when Foretinib is used, it is preferably 5 nM to 5 µM, when ZM323881 is used, it is preferably 0.1 µM to 10 µM, when CP-673451 is used, it is preferably 0.1 µM to 10 µM, when Crenolanib is used, it is preferably 0.1 µM to 10 µM, and when Crizotinib is used, it is preferably 0.1 µM to 10 µM. The concentration is not limited to these concentrations.

The method for culturing the cell population in the aforementioned steps (2) and (2') is the same as that in the above-mentioned (0-2) or (0-3). The culture period is the same, and the culture may be continued at least while the cell population is in contact with the receptor-type tyrosine kinase inhibitor.

### 2. Cell population containing cardiomyocytes

The present invention also provides a cell population containing cardiomyocytes (hereinafter also to be referred to as "the cell population of the present invention") obtained by the production method or purification method of the present invention. As described above, the cell population of the present invention contains cardiomyocytes with high purity. High purity means that the ratio of cardiomyocytes in the cell population (number of cardiomyocytes in the cell population/total number of cells in the cell population) is specifically not less than 80% (e.g., 85%, 90%, 91%, 92%, 93%, 94%, 95%, 96%, 97%, 98%, 99%, or higher). When the above-mentioned cell population and other cells or cell populations such as mesenchymal stem cells and the like are mixed and used, the ratio of the above-mentioned cardiomyocytes is naturally the one before mixing with other cells or cell populations. In a preferred embodiment, the cell population of the present invention contains cardiomyocytes at a higher percentage than a cell population obtained by a conventional method of inducing cardiomyocytes from pluripotent stem cells. Such cell populations may be further purified by cell sorting or the like, and such purified cell population is also encompassed in the "the cell population of the present invention".

### 3. Agent for cell transplantation therapy

The present invention also provides an agent for cell transplantation therapy containing the cell population of the present invention (hereinafter also to be referred to as "the cell transplantation therapy agent of the present invention"). The cell transplantation therapy agent of the present invention may be used for autologous transplantation or allotransplantation. In addition, it may be used in combination with other medicaments such as immunosuppressant. As mentioned above, since the cell population of the present invention contains cardiomyocytes with high purity, it is suitably used as a starting material of an agent for cell transplantation therapy, and the cell population of the present invention and the cell transplantation therapy agent of the present invention are useful for the treatment or prophylaxis of cardiac diseases. Therefore, a method for treating or preventing cardiac diseases, including administering or transplanting an effective amount of the cell population or the cell transplantation therapy agent of the present invention to a mammal (e.g., human, mouse, rat, monkey, bovine, horse, swine, dog, etc.) as the target of treatment or prophylaxis is also encompassed in the present invention. Examples of the cardiac disease to be the target of treatment or prophylaxis include, but are not limited to, diseases such as cardiac failure, ischemic cardiac diseases, myocardial infarction, cardiomyopathy, myocarditis, hypertrophic cardiomyopathy, dilated phase of hypertrophic cardiomyopathy, dilated cardiomyopathy, and the like, deficiency due to disorder, and the like.

From the aspect that rejections do not occur when the cell population of the present invention is used as an agent for cell transplantation therapy, it is desirable to use a cell population containing cells derived from iPS cells established from somatic cells that have the same or substantially the same HLA genotype as the individual who receives transplantation. As used herein, "substantially the same" means that the HLA genotypes match to the extent that an immunosuppressant can suppress an immune response to the transplanted cells. For example, it refers to a somatic cell with an HLA type in which 3 loci of HLA-A, HLA-B, and HLA-DR or 4 loci including HLA-C are matched. In addition, it is also possible to embed the cells in a capsule such as polyethylene glycol or silicon, or a porous container, etc. and transplant the cells while avoiding rejections.

The cell population of the present invention can be produced as a parenteral formulation such as injection, suspension, drip infusion, or the like, in a mixture with a pharmaceutically acceptable carrier, by a conventional means. Examples of the pharmaceutically acceptable carrier that can be contained in the parenteral preparation include aqueous liquids for injection, such as isotonic solution containing physiological saline, glucose, and other auxiliary drugs (e.g., D-sorbitol, D-mannitol, sodium chloride, and the like). The agent for cell transplantation therapy of the present invention may be formulated with, for example, a buffering agent (e.g., phosphate buffer solution, sodium acetate buffer solution), a soothing agent (e.g., benzalkonium chloride, procaine hydrochloride, and the like), a stabilizer (e.g., human serum albumin, polyethylene glycol, and the like), a preservative, an anti-oxidant, or the like. When the agent for transplantation therapy of the present invention is formulated as an aqueous suspension, a cell population containing cardiomyocytes may be suspended in the above-mentioned aqueous solution at about 1×10⁶ to about 1×10⁸ cells/mL. It can also be administered with a scaffolding material that promotes engraftment. Examples of the scaffolding material include, but are not limited to, components of biological origin such as collagen and the like, and synthetic polymers such as polylactic acid and the like that substitute for the components.

Alternatively, cardiac diseases may be treated by adhering a sheet formed from the obtained cardiomyocytes to the heart of patients. When a cardiac muscle sheet is administered, the sheet is disposed to cover the desired area. Here, disposing to cover the desired area can be performed using a technique well known in the pertinent field. During the disposing, when the desired area is large, the sheet may also be placed to surround the tissue. In order to achieve the desired effect, the administration may include several times of disposing on the same area. When disposing is performed several times, it is desirable to secure sufficient time so that the desired cells will be engrafted in the tissue to allow for angiogenesis. The mechanism of such treatment of cardiac diseases may be an effect resulting from the engraftment of a cardiac muscle sheet, or an indirect action not based on cell engraftment (e.g., effect by recruiting recipient-derived cells to damaged site caused by secretion of an attractant). When a cardiac muscle sheet is used in the treatment of cardiac diseases, it may contain a cell scaffolding material (scaffold) such as collagen, fibronectin, laminin, or the like, in addition to cardiomyocytes. Alternatively, it may contain any cell type (possibly in plurality) in addition to cardiomyocytes. The number of cardiomyocytes used for the treatment of cardiac diseases is not particularly limited as long as the cardiac muscle sheet to be administered exhibits an effect in the treatment of cardiac diseases and can be appropriately increased or decreased for adjustment according to the size of the affected part and the size of the body.

In still another embodiment, the cell population of the present invention can also be used for drug screening or drug cardiotoxicity evaluation for the treatment of cardiac disease. For example, by administering a test drug to the cell population of the present invention, and then measuring the response of the cardiomyocytes, the effect and toxicity of the test drug can be evaluated.

While the present invention is further explained specifically in the following Examples, the scope of the present invention is not limited by such Examples.

### [Example]

### Example 1. Single cell RNA sequencing analysis of cells induced to differentiate into cardiomyocytes from iPS cells

A strain for evaluation of a clinical iPS cell line produced by the Center for iPS Cell Research and Application, Kyoto University (CiRA) was used. Maintenance culture of iPS cell line was performed according to the conventional method (Okita K, et al. Stem Cells. 2012 Nov 29. doi: 10.1002/stem.1293). Induction of differentiation into cardiomyocytes was performed according to the method described in a paper (Miki et al, Cell Stem Cell. 2015 Jun 4; 16. doi: 10.1016/j.stem. 2015.04.005.). Cardiomyocytes at 22 days after the start of differentiation induction were subjected to single cell RNA sequence analysis using Chromium manufactured by 10X Genomics. Software (Cellranger, Seurat, Loupe Cell Browser) was used to cluster cells and analyze each cluster. It was found that the cell populations induced to differentiate from iPS cells into cardiomyocytes contained, in addition to the cardiomyocyte population expressing sarcomeric-α-actinin and cardiac Troponin T, three non-cardiomyocyte populations that do not express sarcomeric-α-actinin or cardiac Troponin T (considered to be smooth muscle-like cell (SMC), endothelial-like cell (EC), and endoderm lineage cell (END) from gene expression) (Fig. 1).

### Example 2. Analysis of non-cardiomyocytes present in cell population induced to differentiate into myocardium from iPS cells

The gene expression of receptor-type tyrosine kinases was analyzed by Loupe Cell Browser analysis of single-cell RNA sequencing data. As a result, it was found that the gene expression of receptor-type tyrosine kinases such as PDGFRA, PDGFRB, VEGFR1, VEGFR2, c-Met (HGFR), FGFR4, and the like was high in non-cardiomyocyte populations, but not high in cardiomyocyte populations (Fig. 2).

### Example 3. Staining of non-cardiomyocytes with cell surface markers

A double knock-in human iPS cell line was produced by inserting the reporter protein sequence of EmGFP (SEQ ID NO: 1) into the TNNI1 gene locus and mCherry (SEQ ID NO: 2) into the TNNI3 gene locus. The human iPS cell line was produced using PBMC (LP_167, Sample ID: 20130318) purchased from CTL and an episomal vector (loaded gene; OCT3/4, KLF4, SOX2, L-MYC, LIN28, mouse p53DD)(reference document; Okita K, et al. Stem Cells. 2012 Nov 29. doi: 10.1002/stem.1293).

Maintenance culture of iPS cell line was performed according to the conventional method (Okita K, et al. Stem Cells. 2012 Nov 29. doi: 10.1002/stem.1293).

Induction of differentiation into cardiomyocytes was performed according to the method described in a paper (Miki et al, Cell Stem Cell. 2015 Jun 4; 16. doi: 10.1016/j.stem. 2015.04.005.) and using a 6-well plate. For brief explanation, induction of differentiation into cardiomyocytes was performed by treating the reporter iPS cell line for 4 to 5 min with TrypLE select (Life Technologies) diluted to 1/2 with 0.5 mM EDTA/PBS, detaching the cells with a cell scraper (IWAKI), and dissociating the cells into single cells by pipetting. The medium was removed by centrifugation at 1,000 rpm for 5 min, and the obtained cells were seeded at 2×10⁶ cells per well of a 6-well plate and cultured in StemPro34 medium, supplemented with 1% L-glutamine, transferrin 150 µg/mL, ascorbic acid 50 µg/mL (sigma), monothioglycerol 4×10⁻⁴ M, 10 µM Y-27632, 2 ng/ml BMP4 (R&D), and 0.5% Growth Factor Reduced Matrigel, at 1.5 mL/well under 37°C, 5% oxygen conditions to form embryoid bodies (day 0).

The next day (day 1), 1.5 mL of StemPro34 medium supplemented with 1% L-glutamine, transferrin 150 µg/mL, ascorbic acid 50 µg/mL (sigma), monothioglycerol 4×10⁻⁴ M, 2 ng/ml BMP4 (R&D) activin A 12 ng/ml, bFGF 5 ng/ml, and BMP4 18 ng/ml was added to each well, and the cells were cultured under 37°C, 5% oxygen conditions for two more days.

Successively (day 3), the 6-well plate was stood while tilting to allow embryoid bodies to form sediments, 80 to 90% of the medium was removed, and IMDM (1.5 mL) was added to each well. The well plate was stood while tilting again to allow embryoid bodies to form sediments, 80 to 90% of the medium was removed, and the cells were cultured in StemPro34 medium supplemented with 1% L-glutamine, transferrin 150 µg/mL, ascorbic acid 50 µg/mL (sigma), monothioglycerol 4×10⁻⁴ M, 10 ng/ml VEGF, 1 µM IWP-3, 0.6 µM Dorsomorphin, and 5.4 µM SB431542, under 37°C, 5% oxygen conditions for 3 days.

Successively (day 6), the 6-well plate was stood while tilting to allow embryoid bodies to form sediments, 80 to 90% of the medium was removed, and StemPro34 medium supplemented with 1% L-glutamine, transferrin 150 µg/mL, ascorbic acid 50 µg/mL (sigma), monothioglycerol 4×10⁻⁴ M, and 5 ng/ml VEGF was added. The cells were cultured for 8 days under 37°C, 5% oxygen conditions. During this period, the medium was exchanged with a medium under the same conditions once every 2 to 3 days.

On day 15 of differentiation induction, a cell culture medium containing embryoid bodies was centrifuged at 200 g for 1 min, and the supernatant was removed with an aspirator. PBS was added, and the mixture was centrifuged at 200xg for 1 min, and the supernatant was removed with an aspirator. A solution (3 mL) obtained by adding DNase 10 µg/mL and Liberase 100 µg/mL to IMDM (Iscove's Modified Dulbecco's Media) was added to each tube, and the mixture was stood at 37°C under general oxygen conditions for 1 hr. After 1 hr, the tube was centrifuged at 400 g for 5 min, and the supernatant was removed while avoiding sucking embryoid bodies. A solution (2 mL) obtained by adding DNase 10 µg/mL to Accutase (Thermo) was added to each tube, and the mixture was stood at 37°C under general oxygen conditions for 10 min. After standing, single cells were obtained by pipetting, a medium (2 mL) obtained by adding DNase 10 µg/mL to IMDM was added to each tube, and the mixture was mixed by inversion to prepare a single cell suspension.

The above-mentioned single cell suspension was centrifuged at 200xg for 5 min, the supernatant was removed, and the cells were cryopreserved -80°C.

The cryopreserved cells were thawed in a warm bath at 37°C, centrifuged at 400xg for 5 min, and the supernatant was removed.

After tapping the cell pellets, PBS containing 1% BSA was added by 1 mL, and the mixture was centrifuged at 300xg for 3 min. The supernatant was removed. The cells were stained with APCFire750-labeled anti-CD326 antibody, PE-labeled anti-CD49a antibody, BV605-labeled anti-CD31 antibody, and DAPI in PBS containing 1% BSA. DAPI-positive dead cells were removed, and the separation of cell population and the ratio of each cell population were measured by measuring the signal amount of each fluorescence dye in nuclear cells.

Cardiomyocytes obtained by differentiation induction of the above-mentioned reporter iPS cells into cardiac muscle were separated into 4 main cell populations of CD326-positive cells, CD326-negative CD31-positive cells, CD326-negative CD31-negative CD49a-positive cells, and CD326-negative CD31-negative CD49a-negative cells due to differences in the expression of these three surface markers. The four cell populations consisted of three populations mainly composed of non-cardiomyocytes expressing one or more of these three surface markers (populations containing many cells not expressing cardiac muscle reporter genes and a cardiomyocyte population negative for all three markers (cell population expressing cardiac muscle reporter gene). Not less than 99% of the cell population negative for all three markers were cardiomyocytes (cells expressing cardiac muscle reporter gene) (Fig. 3).

Then, taking note of the receptor-type tyrosine kinase found in Example 2, whether or not cardiomyocytes with relatively low receptor-type tyrosine kinase expression can be purified by using a receptor-type tyrosine kinase inhibitor was verified under various conditions (Experimental Examples 1 to 9).

In the following Experimental Examples 1 to 9, compound A is N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, and compound B is N-{4-[(6,7-dimethoxyquinolin-4-yl)oxy]-3-fluorophenyl}-N'-(4-fluorophenyl)cyclopropane-1,1-dicarboxamide.

### Experimental Example 1. Verification of cardiomyocyte purification action by receptor-type tyrosine kinase inhibitor

### <Induction of differentiation into cardiomyocytes>

As human iPS cell line, a cell line (parent cell line of reporter cell used for staining non-cardiomyocytes with cell surface marker) produced using PBMC (LP_167, Sample ID: 20130318) purchased from CTL and an episomal vector (loaded gene; OCT3/4, KLF4, SOX2, L-MYC, LIN28, mouse p53DD) (reference document; Okita K, et al. Stem Cells. 2012 Nov 29. doi: 10.1002/stem.1293) was used.

Maintenance culture of iPS cell line was performed according to the conventional method (Okita K, et al. Stem Cells. 2012 Nov 29. doi: 10.1002/stem.1293). Induction of differentiation into cardiomyocytes was performed according to the method described in a paper (Miki et al., Cell Stem Cell. 2015 Jun 4; 16. doi: 10.1016/j.stem. 2015.04.005.) and using a 6-well plate. VEGF was not added after Day 8.

On day 8 of differentiation induction, the embryoid bodies were collected in one centrifugal tube, allowed to stand at room temperature for several minutes to cause sedimentation of the embryoid bodies. The supernatant was removed with an aspirator, and a medium was added to replace the medium. The embryoid body suspension was transferred to a 6-well plate, and a 1/100 amount of an evaluation compound (compounds and concentrations of Experiment Nos. 2 to 8 shown in Table 1 below) was added at a concentration of 100 times the final concentration to each well. The plate was stirred to perform cell culture.

**[Table 1]**

| experiment number | compound | final concentration (µM) |
|---|---|---|
| 1 | untreated | - |
| 2 | compound A | 0.5 |
| 3 | AMG337 | 2 |
| 4 | ASP5878 | 0.02 |
| 5 | ASP5878 | 0.05 |
| 6 | ASP5878 | 0.12 |
| 7 | BGJ398 | 0.2 |
| 8 | BGJ398 | 1 |

On day 10 of differentiation induction, the 6-well plate was stood for several minutes while tilting to allow embryoid bodies to form sediments. The supernatant was removed with an aspirator, and a medium was added to replace the medium in each well. Similar to day 8, a 1/100 amount of an evaluation compound was added at a concentration of 100 times the final concentration, whereby the compound was added. On day 13 of differentiation induction, the 6-well plate was tilted to allow embryoid bodies to form sediments, and the embryoid bodies were transferred to a 1.5 mL tube with a pipette provided with a chip for wide-diameter 1 mL pipette. Dissociation into single cells was performed according to the staining of non-cardiomyocytes by cell surface marker, and a single cell suspension was used for the cardiomyocyte marker-positive cell rate measurement, and non-cardiomyocyte marker-positive cell rate measurement.

### <Cardiac muscle marker-positive cell rate (cardiomyocyte rate) measurement>

The above-mentioned single cell suspension was dispensed to a 1.5 mL tube, centrifuged at 400 g for 3 min, and the supernatant was removed. Cell pellets were resuspended in Cytofix/Cytoperm Fixation/Permeabilization Solution (BD), and fixed by allowing to stand at room temperature for 15 min. To each tube was added 1xPerm/Wash Buffer (1 mL), and the mixture was centrifuged at 1500 g for 3 min, and the supernatant was removed to wash the cells. After tapping the cell pellets, 1xPerm/Wash Buffer (1 mL) was added, and a similar operation was performed to wash the cells. After tapping the cell pellets, the cell pellets were stained with fluorescence dye Alexa647 using an anti-sarcomeric α-actinin antibody in 1xPerm/Wash Buffer containing 1% BSA, and DNA staining was further performed using DAPI (4',6-Diamidino-2-phenylindole Dihydrochloride). The Sarcomeric α-actinin-positive cell rate was analyzed by measuring the fluorescence signal amount of Alexa647 in the cell population excluding dead cells (cells with DNA amount of Sub-G1) by using a flow cytometer. As a result, the cardiomyocyte rate (Actinin-positive cell rate) increased by the treatment with compound A (N-[5-({2-[(cyclopropanecarbonyl)amino]imidazo[1,2-b]pyridazin-6-yl}oxy)-2-methylphenyl]-1,3-dimethyl-1H-pyrazole-5-carboxamide), AMG337, ASP5878, or BGJ398, as compared with no treatment with compound (Fig. 4).

### <Non-cardiomyocytes marker-positive cell rate (non-cardiomyocyte rate) measurement>

Using an unfrozen single cell suspension, the staining, measurement, and analysis were performed according to the staining of non-cardiomyocytes by cell surface marker. As a result, the non-cardiomyocyte rate (rate of cells positive to any of CD326, CD31, CD49a) decreased by the treatment with compound A, AMG337, ASP5878, or BGJ398, as compared with no treatment with compound (Fig. 5).

### Experimental Example 2. Verification of cardiomyocyte purification action by receptor-type tyrosine kinase inhibitor

### <Induction of differentiation into cardiomyocytes>

As human iPS cells, the same cell line as in Experimental Example 1 was used, and differentiation induction into cardiomyocytes was also performed by a similar method. The medium was changed on day 8, day 10, and day 13 of the differentiation induction, and it was performed by a method similar to that in Experimental Example 1 on day 8 and day 10 of the differentiation induction, and performed by a method similar to that for day 10 in Experimental Example 1 on day 13 of the differentiation induction. On day 8, day 10, and day 13 of the differentiation induction, evaluation compounds (compounds and concentrations of Experiment Nos. 2, 3 shown in Table 2 below) were added by a method similar to that in Experimental Example 1. ASP5878 in experiment number 3 was added only on day 8 and day 10 of the differentiation induction. Respective experiments were performed using 4 wells each.

**[Table 2]**

| experiment number | compound | final concentration (µM) |
|---|---|---|
| 1 | untreated | - |
| 2 | Foretinib | 2 |
| 3 | ASP5878 | 0.02 |

On day 16 of differentiation induction, dissociation into single cells, fixation, and cardiomyocyte rate (sarcomeric α-actinin-positive cell rate) measurement were performed by methods similar to those in Experimental Example 1. As a result, the cardiomyocyte rate increased by the treatment with Foretinib, or ASP5878, as compared with no treatment with compound (Fig. 6).

### Experimental Example 3. Verification of cardiomyocyte purification action by receptor-type tyrosine kinase inhibitor

### <Induction of differentiation into cardiomyocytes>

As human iPS cell line, a cell line produced using PBMC (LP_140, Sample ID: 20120808) purchased from CTL and an episomal vector (loaded gene; OCT3/4, KLF4, SOX2, L-MYC, LIN28, mouse p53DD) (reference document; Okita K, et al. Stem Cells. 2012 Nov 29. doi: 10.1002/stem.1293) was used.

Maintenance culture of iPS cell line was performed according to the conventional method (Okita K, et al. Stem Cells. 2012 Nov 29. doi: 10.1002/stem.1293). Induction of differentiation into cardiomyocytes was performed by a method similar to that in Example 1. VEGF was not added after Day 10.

The medium was changed on day 8, day 10, and day 13 of the differentiation induction, and it was performed by a method similar to that in Experimental Example 1 on day 8 and day 10 of the differentiation induction, and performed by a method similar to that for day 10 in Experimental Example 1 on day 13 of the differentiation induction. On day 8, day 10, and day 13 of the differentiation induction, evaluation compounds (compounds and concentrations of Experiment Nos. 2 to 6 shown in Table 3 below) were added by a method similar to that in Experimental Example 1.

**[Table 3]**

| experiment number | compound | final concentration (µM) |
|---|---|---|
| 1 | untreated | - |
| 2 | compound A | 0.5 |
| 3 | Foretinib | 2 |
| 4 | ZM323881 | 2 |
| 5 | Crenolanib | 1 |
| 6 | Crizotinib | 2 |

On day 17 of differentiation induction, dissociation into single cells and non-cardiomyocyte rate (rate of cells positive to any of CD326, CD31, CD49a) measurement were performed by methods similar to those in Experimental Example 1. As a result, the non-cardiomyocyte rate decreased by the treatment with compound A, Foretinib, ZM323881, Crenolanib, or Crizotinib, as compared with no treatment with compound (Fig. 7) .

### Experimental Example 4. Verification of cardiomyocyte purification action by receptor-type tyrosine kinase inhibitor

### <Induction of differentiation into cardiomyocytes>

As human iPS cells, the same cell line as in Experimental Example 3 was used, and differentiation induction into cardiomyocytes was also performed by a similar method.

The medium was changed on day 8, day 10, and day 13 of the differentiation induction, and it was performed by a method similar to that in Experimental Example 1 on day 8 and day 10 of the differentiation induction, and performed by a method similar to that for day 10 in Experimental Example 1 on day 13 of the differentiation induction. On day 8, day 10, and day 13 of the differentiation induction, evaluation compounds (compounds and concentrations of Experiment Nos. 2, 3 shown in Table 4 below) were added by a method similar to that in Experimental Example 1. Respective experiments were performed using 4 wells each.

**[Table 4]**

| experiment number | compound | final concentration (µM) |
|---|---|---|
| 1 | untreated | - |
| 2 | compound A | 0.5 |
| 3 | Foretinib | 0.5 |

On day 17 of differentiation induction, dissociation into single cells, cell count measurement, and cardiomyocyte rate measurement were performed. Fixation and cardiomyocyte rate (sarcomeric α-actinin-positive cell rate) measurement were performed by methods similar to those in Experimental Example 1. As a result, the cardiomyocyte rate increased by the treatment with compound A and Foretinib, as compared with no treatment (Fig. 8).

The above-mentioned single cell suspension was 5-fold diluted by adding to IMDM medium prepared in a 1.5 mL tube, and mixed by inversion, and the cell count was measured with a cell counter NC-200 (Chemometec). As a result, about 70% of the number of the cells was recovered by the treatment with Foretinib and almost the same number of cells was recovered by the treatment with compound A, as compared with no treatment (Fig. 9).

### Experimental Example 5. Verification of cardiomyocyte purification action by receptor-type tyrosine kinase inhibitor

### <Induction of differentiation into cardiomyocytes>

As human iPS cells, the same cell line as in Experimental Example 3 was used, and differentiation induction into cardiomyocytes was also performed by a similar method.

The medium was changed on day 8, day 10, and day 13 of the differentiation induction, and it was performed by a method similar to that in Experimental Example 1 on day 8 and day 10 of the differentiation induction, and performed by a method similar to that for day 10 in Experimental Example 1 on day 13 of the differentiation induction. On day 8, day 10, and day 13 of the differentiation induction, evaluation compounds (compounds and concentrations of Experiment Nos. 2 to 4 shown in Table 5 below) were added by a method similar to that in Experimental Example 1. ASP5878 in experiment number 4 was added only on day 8 and day 10 of the differentiation induction. Respective experiments were performed using 4 wells each.

**[Table 5]**

| experiment number | compound | final concentration (µM) |
|---|---|---|
| 1 | untreated | - |
| 2 | Crenolanib | 1 |
| 3 | compound B | 2 |
| 4 | ASP5878 | 0.02 |

On day 16 of differentiation induction, dissociation into single cells, fixation, and measuring cardiomyocyte rate (sarcomeric α-actinin-positive cell rate) were performed by methods similar to those in Experimental Example 1. As a result, the cardiomyocyte rate (sarcomeric α-actinin-positive cell rate) increased by the treatment with Crenolanib, compound B (N-{4-[(6,7-dimethoxyquinolin-4-yl)oxy]-3-fluorophenyl}-N'-(4-fluorophenyl)cyclopropane-1,1-dicarboxamide), or ASP5878, as compared with no treatment with compound (Fig. 10).

Using the above-mentioned single cell suspension, the cell count was measured by the method described in Experimental Example 4. As a result, the number of recovered cells did not change much depending on the presence or absence of the compound treatment (Fig. 11).

### Experimental Example 6. Verification of cardiomyocyte purification action by receptor-type tyrosine kinase inhibitor

### <Induction of differentiation into cardiomyocytes>

A clinical iPS cell line produced by CiRA was used. Maintenance culture of iPS cell line was performed according to the conventional method (Okita K, et al. Stem Cells. 2012 Nov 29. doi: 10.1002/stem.1293). Induction of differentiation into cardiomyocytes was performed according to the method described in a paper (Miki et al, Cell Stem Cell. 2015 Jun 4; 16. doi: 10.1016/j.stem. 2015.04.005.).

The medium was changed on days 8, 10, 14, 17, and 21 of the differentiation induction, and it was performed by a method similar to that in Experimental Example 1 on day 8 and day 10 of the differentiation induction, and performed by a method similar to that for day 10 in Experimental Example 1 on day 14 and thereafter of the differentiation induction.

On day 14, day 17, and day 21 of the differentiation induction, compound A which is one of the evaluation compounds was added by a method similar to that in Experimental Example 1. Respective experiments were performed using 4 wells each.

**[Table 6]**

| experiment number | compound | final concentration (µM) |
|---|---|---|
| 1 | untreated | - |
| 2 | compound A | 0.2 |

Dissociation in to single cells, fixation, cardiomyocyte rate measurement, and non-cardiomyocyte rate measurement were performed by methods similar to those in Experimental Example 1, and cell count measurement was performed by a method similar to that in Experimental Example 4. As a result, the cardiomyocyte rate increased (Fig. 12) and the non-cardiomyocyte rate decreased (Fig. 13) by the treatment with compound A, as compared with no treatment with compound. The number of recovered cells did not change much depending on the presence or absence of the compound treatment (Fig. 14).

### Experimental Example 7. Verification of cardiomyocyte purification action by receptor-type tyrosine kinase inhibitor

### <Induction of differentiation into cardiomyocytes>

As human iPS cells, a clinical iPS cell line produced by CiRA was used. Using the same cell line as in Experimental Example 6, differentiation induction into cardiomyocytes was also performed by a similar method.

The medium was changed on day 8, day 10, and day 13 of the differentiation induction, and it was performed by a method similar to that in Experimental Example 1 on day 8 and day 10 of the differentiation induction, and performed by a method similar to that for day 10 in Experimental Example 1 on day 13 of the differentiation induction. On day 8, day 10, and day 13 of the differentiation induction, evaluation compounds (compounds and concentrations of Experiment Nos. 2 to 9 shown in Table 7 below) were added by a method similar to that in Experimental Example 1 (two wells were used for experiment number 1, and one well was used for others).

**[Table 7]**

| experiment number | compound | final concentration (µM) |
|---|---|---|
| 1 | untreated | - |
| 2 | compound A | 0.5 |
| 3 | Foretinib | 2 |
| 4 | Foretinib | 0.5 |
| 5 | ZM323881 | 2 |
| 6 | CP-673451 | 1 |
| 7 | CP-673451 | 0.3 |
| 8 | Crizotinib | 2 |
| 9 | Crizotinib | 0.5 |

On day 17 of differentiation induction, dissociation into single cells, fixation, cardiomyocyte rate (sarcomeric α-actinin-positive cell rate) measurement, and non-cardiomyocyte rate measurement were performed by methods similar to those in Experimental Example 1. The cardiomyocyte rate increased (Fig. 15) and the non-cardiomyocyte rate decreased (Fig. 16) by the treatment with compound A, Foretinib, ZM323881, or CP-67351, Crizotinib, as compared with no treatment with compound (Fig. 16) .

### Experimental Example 8. Verification of cardiomyocyte purification action by receptor-type tyrosine kinase inhibitor

### <Induction of differentiation into cardiomyocytes>

As human iPS cells, the same cell line as in Experimental Example 6 was used, and differentiation induction into cardiomyocytes was also performed by a similar method.

The medium was changed on days 8, 10, 13, 17, and 20 of the differentiation induction, and it was performed by a method similar to that in day 8 in Experimental Example 1 on day 8 of the differentiation induction, and performed by a method similar to that for day 10 in Experimental Example 1 on day 10 and thereafter of the differentiation induction. On days 8, 10, 13, 17, and 20 of the differentiation induction, an evaluation compound Crenolanib was added according to the conditions described in Table 8 and by a method similar to that in Experimental Example 1. Compounds were added on or after day 8 of differentiation induction in experiment number 4, on or after day 10 of differentiation induction in experiment numbers 2 and 3, and on or after day 13 of differentiation induction in experiment number 5. Four wells were used for experiment number 1, three wells were used for experiment numbers 2 and 3, and one well was used for experiment numbers 4 and 5.

**[Table 8]**

| experiment number | compound | final concentration (µM) | date of start of addition |
|---|---|---|---|
| 1 | untreated | - | - |
| 2 | Crenolanib | 0.2 | day 10 |
| 3 | Crenolanib | 1 | day 10 |
| 4 | Crenolanib | 1 | day 8 |
| 5 | Crenolanib | 1 | day 13 |

On day 23 of differentiation induction, dissociation into single cells, fixation, cardiomyocyte rate (sarcomeric α-actinin-positive cell rate) measurement, and non-cardiomyocyte rate measurement were performed by methods similar to those in Experimental Example 1. The cardiomyocyte rate increased (Fig. 17) and the non-cardiomyocyte rate decreased (Fig. 18) by the treatment with Crenolanib.

### Experimental Example 9. Verification of cardiomyocyte purification action by receptor-type tyrosine kinase inhibitor

### <Induction of differentiation into cardiomyocytes>

As human iPS cells, the same cell line as in Experimental Example 6 was used, and differentiation induction into cardiomyocytes was also performed by a similar method.

The medium was changed by a method similar to that in day 8 in Experimental Example 1 on day 8 of the differentiation induction, and performed by a method similar to that for day 10 in Experimental Example 1 on day 10 and day 13 of the differentiation induction. On days 8, 10, and 13 of the differentiation induction, evaluation compounds (compounds and concentrations of Experiment Nos. 2, 3 shown in Table 9 below) were added by a method similar to that in Experimental Example 1. Respective experiments were performed using 4 wells each.

**[Table 9]**

| experiment number | compound | final concentration (µM) |
|---|---|---|
| 1 | untreated | - |
| 2 | Foretinib | 0.5 |
| 3 | Crizotinib | 2 |

On day 17 of differentiation induction, dissociation into single cells, fixation, cardiomyocyte rate (sarcomeric α-actinin-positive cell rate) measurement, and non-cardiomyocyte rate measurement were performed by methods similar to those in Experimental Example 1, and cell count measurement was performed by a method similar to that in Experimental Example 4. The cardiomyocyte rate increased (Fig. 19) and the non-cardiomyocyte rate decreased (Fig. 20) by the treatment with Foretinib or Crizotinib, as compared with no treatment with compound. About 80% of the number of cells was recovered with Crizotinib and almost the same number of cells was recovered by the treatment with Foretinib, as compared with no treatment with compound (Fig. 21).

From the above, it was shown that cardiomyocytes in embryoid bodies can be purified in various cell lines by a convenient treatment of only adding various kinds of receptor-type tyrosine kinase inhibitors to a medium.

The receptor-type tyrosine kinases to be the targets of the compounds used in the above-mentioned Experimental Examples 1 to 9 are shown in Table 10.

**[Table 10]**

| compound | compound A | AMG337 | ASP5878 | BGJ398 | Foretinib |
|---|---|---|---|---|---|
| target | VEGFR1,2,3/ PDGFRα,β | c-Met (HGFR) | FGFR1-4 | FGFR1-3 | VEGFR1,2,3/ c-Met (HGFR) |
| compound | ZM323881 | CP-673451 | Crenolanib | Crizotinib | compound B |
| target | VEGFR2 | PDGFRα,β | PDGFRα,β | c-Met | c-Met(HGFR) |

### [Industrial Applicability]

According to the present invention, a cell population containing cardiomyocytes at a high purity is provided. Such cell population is useful because it can be preferably used for cell transplantation therapy for cardiac diseases and screening for therapeutic agents for cardiac diseases.

This application is based on a patent application No. 2020-050268 filed in Japan (filing date: March 19, 2020), the contents of which are incorporated in full herein.

## Claims

1. A method for producing a cell population comprising cardiomyocytes, comprising
(1) a step of bringing a receptor-type tyrosine kinase inhibitor (excluding EGF receptor inhibitors) into contact with a cell population containing cardiomyocytes or cardiac progenitor cells, and other cells, the cell population being obtained by culturing pluripotent stem cells in a medium for cardiomyocyte differentiation, and
(2) a step of culturing the cell population.

2. The method according to claim 1, wherein the cell population is contacted with the receptor-type tyrosine kinase inhibitor in the step (1) on or after 4 days from the start of differentiation induction of pluripotent stem cells.

3. The method according to claim 1 or 2, wherein the cell population is contacted with the receptor-type tyrosine kinase inhibitor for not less than one day in the step (1).

4. The method according to any one of claims 1 to 3, wherein the inhibitor is at least one inhibitor against a receptor-type tyrosine kinase selected from the group consisting of a VEGF receptor, a PDGF receptor, an HGF receptor, and an FGF receptor.

5. The method according to any one of claims 1 to 4, wherein the inhibitor is at least one member selected from the group consisting of N-[5-({2-[(cyclopropanecarbonyl)amino]imidazo[1,2-b]pyridazin-6-yl}oxy)-2-methylphenyl]-1,3-dimethyl-1H-pyrazole-5-carboxamide, N-{4-[(6,7-dimethoxyquinolin-4-yl)oxy]-3-fluorophenyl}-N'-(4-fluorophenyl)cyclopropane-1,1-dicarboxamide, AMG337, ASP5878, BGJ398, Foretinib, ZM323881, CP-673451, Crenolanib, and Crizotinib.

6. The method according to any one of claims 1 to 5, wherein the pluripotent stem cell is an induced pluripotent stem cell.

7. A cell population containing cardiomyocytes, obtained by the method according to any one of claims 1 to 6.

8. An agent for cell transplantation therapy, comprising the cell population according to claim 7.

9. A method for purifying cardiomyocytes, comprising
(1) a step of bringing a receptor-type tyrosine kinase inhibitor into contact with a cell population containing cardiomyocytes or cardiac progenitor cells, and other cells, the cell population being obtained by culturing pluripotent stem cells in a medium for cardiomyocyte differentiation, and
(2) a step of culturing the cell population.
